# FASCICULE DE BREVET EUROPEEN

(11) **EP 3 223 775 B1**
(45) Date de publication et mention de la délivrance du brevet: **23.12.2020**
(21) Numéro de dépôt: 15798459.2
(22) Date de dépôt: 24.11.2015
(51) Int. Cl.: A61K 8/25, B01F 17/00, A61K 8/06, A61K 8/04, A61Q 19/00, A61K 8/02

(54) **GEL AQUEUX OU HYDROALCOOLIQUE DE PHYLLOSILICATES SYNTHETIQUES A TITRE D'AGENT VISCOSANT, MATIFIANT ET/OU HOMOGENEISANT D'APPLICATION**
HYDROALKOHOLISCHES ODER WÄSSRIGES GEL AUS SYNTHETISCHEN PHYLLOSILIKATEN ALS VERDICKUNGS-, MATTIERUNGS- UND/ODER ANWENDUNGSHOMOGENISIERUNGSMITTEL
HYDROALCOHOLIC OR AQUEOUS GEL OF SYNTHETIC PHYLLOSILICATES AS A THICKENING, MATTIFYING AND/OR APPLICATION HOMOGENISING AGENT

(30) Priorité: 24.11.2014 FR 1461334
(43) Date de publication de la demande: 04.10.2017
(73) Titulaire: L'Oréal, 75008 Paris (FR)
(72) Inventeur: BOUARFA, Bouchra, 94550 Chevilly La Rue (FR); FERRARI, Véronique, 94550 Chevilly La Rue (FR); SPRINGINSFELD, Fabrice, 94550 Chevilly La Rue (FR); LORANT, Raluca, 94550 Chevilly La Rue (FR); SEBILLOTTE-ARNAUD, Laurence, 94550 Chevilly La Rue (FR); CHABRILLANGEAS, Mathieu, 94550 Chevilly La Rue (FR)
(74) Mandataire: Nony
(86) Numéro de dépôt international: PCT/EP2015/077519
(87) Numéro de publication internationale: WO 2016/083385

(56) Documents cités:
- WO-A2-2008/009799
- DE-A1-102011 012 214
- US-A- 4 830 843
- US-A1- 2014 205 528
- Record ET AL: "Smoothing Cleanser Product Description", , 1 octobre 2014 (2014-10-01), XP055194845, Extrait de l'Internet: URL:http://www.gnpd.com/sinatra/recordpage /2759483/from_search/aK4TJxniUk/ [extrait le 2015-06-10]
- Record ET AL: "Extra Cool Shower Gel Product Description", , 1 juillet 2014 (2014-07-01), XP055194851, Extrait de l'Internet: URL:http://www.gnpd.com/sinatra/recordpage /2554717/from_search/9NunafB0Es/ [extrait le 2015-06-10]
- Record ET AL: "Waterproof Gel Eyeliner Product Description", , 1 août 2014 (2014-08-01), XP055194864, Extrait de l'Internet: URL:http://www.gnpd.com/sinatra/recordpage /2475979/from_search/WlYM8YLaqB/ [extrait le 2015-06-10]

## Description

La présente invention concerne le domaine des produits cosmétiques plus particulièrement dédiés au soin et/ou au maquillage de la peau, des ongles et des lèvres et vise notamment à proposer des compositions présentant des performances accrues en termes de matité, de viscosité et/ou d'homogénéité après application et avantageusement donnant satisfaction simultanément à l'ensemble de ces propriétés.

Une grande partie des consommateurs de produits cosmétiques dédiés au soin ou maquillage de la peau, des lèvres, voire des ongles, est aujourd'hui dans l'attente de produits qui soient aptes à procurer un effet en termes de matité et d'homogénéité après application qui soit amélioré car susceptible d'être alors perçu comme un résultat naturel. Cette attente est notamment requise d'une manière générale à l'égard des produits du soin, mais également désormais pour certains produits dédiés au maquillage. Par ailleurs, ces mêmes utilisateurs maintiennent leurs exigences usuelles au niveau de ces produits cosmétiques à savoir consistance agréable et facile à manipuler, « play-time » ou encore qualités d'étalement acceptables en termes de ressenti sensoriel et de durée, des propriétés qui relèvent d'un aspect plutôt rhéologique.

Pour des raisons évidentes, satisfaire l'ensemble de ces exigences n'est pas immédiat pour le formulateur de produits cosmétiques.

Par exemple, une viscosité satisfaisante sur le plan de la consistance peut être en revanche préjudiciable en termes de résultat d'homogénéité après l'application. Or, un résultat inhomogène aura une incidence sur la qualité du dépôt. En maquillage, il peut se traduire par exemple par une répartition inhomogène des pigments et colorants. De même, il peut également être préjudiciable pour un produit de soin véhiculant des actifs à l'image par exemple des filtres UV. Pour des raisons évidentes, il est important que la formule de soin soit répartie de façon homogène sur la peau pour faciliter l'action de ces actifs.

De même, une performance vérifiée effectivement sur une peau normale, par exemple en termes de matité et d'homogénéité après application, peut ne pas être reproduite sur une peau grasse. En effet, la sécrétion importante de sébum observée sur une peau grasse a pour conséquence d'engendrer une brillance cutanée non désirable. Sur de telles peaux, un résultat homogène et naturel, et donc généralement mat, procuré par un produit de soin ou de maquillage, a malheureusement tendance à se dégrader visuellement au cours de la journée. La matification initiale de même que l'effet homogène sont ainsi altérés.

En conséquence, il est difficile de disposer aujourd'hui d'un produit cosmétique de soin ou de maquillage, d'une part, donnant satisfaction à toutes ses attentes et, d'autre part, dont les performances puissent être vérifiées sur un nombre très large de consommateurs. Ainsi, parmi ces consommateurs, figurent également les utilisateurs dont la peau présente des rides, des ridules ou des pores. Le résultat homogène et naturel attendu par ces utilisateurs requiert également le plus souvent une diminution de la perception du relief de la surface traitée ou maquillée.

Pour répondre à ces différentes attentes des utilisateurs, il est classiquement mis en œuvre dans les compositions cosmétiques plusieurs natures de charges minérales ou organiques. En effet, selon leur nature chimique et leurs propriétés physico-chimiques, elles permettent de cibler plus particulièrement certaines attentes des consommateurs. Par exemple, les charges présentant des propriétés optiques de diffusion de la lumière connues sous le nom d'effet soft-focus (ou effet de flou) permettent de lisser optiquement le microrelief et de camoufler les imperfections de la peau. D'autres charges connus pour leur aptitude à absorber le sébum et la transpiration sont privilégiées pour garantir un effet matifiant dans le temps.

A titre représentatif des charges les plus utilisées, peuvent notamment être citées les charges spécifiques à l'image des particules de silice hydrophobe (aérogels de silice hydrophobe) comme décrit dans WO2013/190112, WO2013/160362, WO13190104, ou dans WO13194100, ou des silices fonctionnalisées par des groupements anioniques comme décrit dans US2005/0192366. Peuvent également être cités certains micas enrobés de matériaux inorganiques et/ou de polyméthacrylate de méthyle (PMMA), l'amidon, des poudres de nylon, les poudres de polyéthylène, la poly-bêta-alanine, les poudres de poly(méth)acrylate de méthyle, le nitrure de bore, ou encore le talc naturel de granulométrie (ou taille moyenne des particules) 1,8 micron ou la perlite.

Toutefois, aucune de ces charges ne permet de satisfaire l'ensemble des exigences précitées. En outre, la mise en œuvre de certaines de ces charges peut générer des effets annexes indésirables dans certaines formulations cosmétiques.

Ainsi les poudres de nylon, de PMMA, de nitrure de bore, de talc naturel ou de l'aérogel de silice sont difficiles à disperser en milieu aqueux et peuvent conduire à un toucher rêche. La perlite a tendance, après décantation, à former un dépôt difficile à ré-homogénéiser lorsqu'elle est introduite dans des solutions aqueuses. Quant au talc d'origine naturelle, il présente le risque de contenir de l'amiante ou des métaux lourds, ce qui n'est pas favorable dans un environnement cosmétique. D'autre part, son broyage à partir de bloc naturel ne permet pas de contrôler sa pureté et la taille des particules obtenues. De plus, il est hydrophobe, ce qui ne favorise pas sa dispersion dans des compositions aqueuses.

Il subsiste donc le besoin d'un matériau permettant de satisfaire l'ensemble des exigences précitées.

En particulier, il demeure le besoin d'un matériau qui, formulé dans un produit cosmétique, procure à ce dernier une homogénéité améliorée après application sans affecter ses autres performances notamment en termes de qualité rhéologique et de ressenti sensoriel.

Il subsiste également le besoin d'un matériau doté de performances matifiantes et soft focus, et dont l'effet puisse être vérifié et ceci de manière prolongé dans le temps sur une grande diversité de natures de peaux, par exemple normales, grasses, mais également ridées.

Il subsiste également le besoin de disposer d'un matériau doté de propriétés satisfaisantes en termes de dispersion, pour permettre sa formulation de manière homogène et stabilisée dans l'ensemble des architectures usuellement considérées dans le domaine du soin et du maquillage, tout en n'altérant pas significativement les autres propriétés rhéologiques attendues.

Contre toute attente, les inventeurs ont constaté que la mise en œuvre d'un phyllosilicate synthétique de formule Mg₃Si₄O₁₀(OH)₂ sous la forme d'un gel aqueux ou hydroalcoolique permet précisément de donner satisfaction à l'ensemble de ces attentes.

Des matériaux de type phyllosilicate synthétique selon l'invention sont connus de l'art antérieur, et sont notamment décrits dans les demandes de brevet FR 2 969 594 et FR 2 925 529. Conviennent tout particulièrement à l'invention des phyllosilicates synthétiques tels que ceux décrits dans la demande WO2008/009799 et avantageusement ceux divulgués dans la demande FR 2 977 580.

Toutefois, aucun de ces documents ne considère la valorisation de ces phyllosilicates synthétiques ainsi obtenus dans des compositions relevant d'applications cosmétiques, dermatologiques ou pharmaceutiques.

Ainsi, l'invention décrit l'utilisation d'un gel aqueux ou hydroalcoolique de phyllosilicate synthétique de formule Mg₃Si₄O₁₀(OH)₂ dans une composition cosmétique.La présente invention concerne, selon un premier de ses aspects, un procédé de préparation d'une composition cosmétique, dans lequel un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å est incorporé dans la phase aqueuse de ladite composition. Selon un mode de réalisation, un gel aqueux ou hydroalcoolique de phyllosilicate synthétique selon l'invention est mis en œuvre à titre d'agent viscosant.

L'invention concerne ainsi, selon un autre de ses aspects, l'utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent viscosant dans une composition cosmétique, ledit gel aqueux ou hydroalcoolique de phyllosilicate synthétique étant incorporé dans la phase aqueuse de ladite composition.

Selon un autre mode de réalisation, un gel aqueux ou hydroalcoolique de phyllosilicate synthétique selon l'invention est mis en œuvre à titre d'agent matifiant.

L'invention concerne ainsi, selon un autre de ses aspects, l'utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent matifiant dans une composition cosmétique, ledit gel aqueux ou hydroalcoolique de phyllosilicate synthétique étant incorporé dans la phase aqueuse de ladite composition.

Selon encore un autre mode de réalisation, un gel aqueux ou hydroalcoolique de phyllosilicate synthétique selon l'invention est mis en œuvre à titre d'agent homogénéisant après application, ou homogénéisant d'application.

L'invention concerne ainsi, selon un autre de ses aspects, l'utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent homogénéisant d'application dans une composition cosmétique, ledit gel aqueux ou hydroalcoolique de phyllosilicate synthétique étant incorporé dans la phase aqueuse de ladite composition.

Selon un autre mode de réalisation, un gel aqueux ou hydroalcoolique selon l'invention est mis en œuvre à titre d'agent viscosant, d'agent matifiant et d'agent homogénéisant d'application.

En outre, les inventeurs ont également observé qu'un gel aqueux ou hydroalcoolique selon l'invention permet de procurer à une composition le comprenant des propriétés améliorées de soft focus, c'est-à-dire un effet correcteur des imperfections cutanées amélioré.

Un tel gel permet en outre avantageusement de conférer un résultat naturel après application à la composition le comprenant.

Il a également été observé une amélioration de la mouillabilité d'une composition comprenant un gel selon l'invention.

Selon un autre de ses aspects, la présente invention concerne une composition, notamment cosmétique, comprenant au moins un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å et au moins un ingrédient additionnel, distinct dudit gel de phyllosilicate synthétique, choisi parmi les corps gras siliconés tels que les huiles, les gommes et les cires de silicone; les corps gras non siliconés tels que les huiles, les pâteux et les cires d'origine végétale, minérale, animale et/ou synthétique; les acides gras ayant de 8 à 32 atomes de carbone; les esters et les éthers de synthèse; les hydrocarbures linéaires ou ramifiés, d'origine minérale ou synthétique; les alcools gras ayant de 8 à 26 atomes de carbone; les alcools en C₂-C₆; les glycols; les tensioactifs ; les gélifiants aqueux ou huileux ; les actifs cosmétiques ; les parfums ; les charges ; les matières colorantes ; les vitamines ; les conservateurs ; les polymères filmogènes (tenseurs ou non) ; et leurs mélanges, ladite composition contenant une phase aqueuse formée au moins en partie par ledit gel aqueux ou hydroalcoolique de phyllosilicate synthétique.

Avantageusement, une composition selon l'invention, comprenant ledit gel aqueux ou hydroalcoolique de phyllosilicate synthétique, présente une bande d'absorption infrarouge à 7 200 cm⁻¹, correspondant à la vibration d'élongation attribuée aux groupes silanols Si-OH en bordure des feuillets du phyllosilicate synthétique de l'invention.

Avantageusement, une composition selon l'invention est caractérisée par une absence de bande d'absorption à 7 156 cm⁻¹. Cette bande correspond à la bande de vibration de Mg₂FeOH.

Une composition selon la présente invention présente également, de préférence, une bande d'absorption infrarouge à 7 184 cm⁻¹ correspondant à la vibration d'élongation 2v Mg₃OH.

Il est à noter qu'en présence d'eau adsorbée, par exemple résiduelle, une bande d'absorption infrarouge est détectable, facilement identifiable, par exemple de 5 500 cm⁻¹.

Selon une variante de réalisation, la composition selon l'invention est une composition cosmétique ou dermatologique comprenant un milieu physiologiquement acceptable.

Selon une variante de réalisation, une composition selon l'invention peut avantageusement comprendre, à titre d'ingrédient additionnel, au moins un composé choisi parmi les charges lipophiles, les pigments, notamment hydrophobes, les polymères hydrophobes, les huiles apolaires ou siliconés, et leurs mélanges.

La présence d'un tel ingrédient additionnel s'avère en effet particulièrement intéressante lorsque la composition selon l'invention vise à procurer un effet matifiant.

Selon un autre de ses aspects, l'invention concerne un procédé cosmétique de maquillage et/ou de soin de la peau et/ou des ongles comprenant au moins une étape d'application sur ladite peau et/ou lesdits ongles d'une composition comprenant, dans un milieu physiologiquement acceptable, au moins un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, ladite composition comprenant une phase aqueuse formée au moins en partie par ledit gel aqueux ou hydroalcoolique de phyllosilicate synthétique. Une telle composition peut notamment être telle que décrite plus loin dans le présent texte.

Par « peau », on entend la peau du visage et/ou du corps et les semi-muqueuses (lèvres). De préférence, il s'agira de la peau du visage et/ou du corps et/ou des lèvres.

Pour des raisons évidentes, une composition selon l'invention destinée à être appliquée sur les ongles s'entend également d'une composition destinée à être appliquée sur les faux-ongles, dans la mesure où les effets cosmétiques recherchés sont bien souvent identiques.

Selon une variante préférée, ledit procédé est dédié à procurer un résultat maquillage matifiant et/ou homogène du teint de la peau.

De préférence, la peau, et mieux la peau du visage, peut être figurée par une peau grasse ou luisante.

### PHYLLOSILICATE SYNTHETIQUE SELON L'INVENTION

Le phyllosilicate synthétique conforme à l'invention présente une structure cristalline conforme à celle d'un silicate de magnésium hydroxylé de formule moléculaire Mg₃Si₄O₁₀(OH)₂ appartenant à la famille chimique des phyllosilicates.

Ces phyllosilicates sont généralement constitués par un empilement de feuillets élémentaires de structure cristalline, dont le nombre varie de quelques unités à quelques dizaines d'unités. Chaque feuillet élémentaire est constitué par l'association de deux couches de tétraèdres, dans lesquels se positionnent les atomes de silicium, situées de part et d'autre d'une couche d'octaèdres dans lesquels se positionnent les atomes de magnésium. Ce groupe correspond aux phyllosilicates 2/1, également qualifiées de type T.O.T. (tétraèdre-octaèdre-tétraèdre).

Comme exposé ci-avant, un phyllosilicate synthétique conforme à l'invention peut être obtenu selon un procédé de préparation tel que celui décrit dans la demande WO2008/009799 et est préférentiellement obtenu selon la technologie décrite dans la demande FR 2 977 580.

Ce procédé de préparation comprend notamment un traitement hydrothermal prolongé, qui permet d'obtenir un gel aqueux de phyllosilicate synthétique. Ainsi, le phyllosilicate synthétique selon l'invention est mis en œuvre sous forme d'un gel aqueux ou hydroalcoolique, notamment à l'image de celui directement obtenu à l'issue du procédé de synthèse.

Comme décrit dans la demande FR 2 977 580, les paramètres qui influencent la synthèse et les propriétés d'un phyllosilicate synthétique sous forme de gel convenant à l'invention sont la nature du traitement thermique (200 °C à 900 °C), la pression, la nature des réactifs et leurs proportions.

Plus particulièrement, la durée et la température du traitement hydrothermal permettent de contrôler la taille des particules. Par exemple, plus la température est faible, plus les particules synthétisées sont petites comme décrit dans la demande FR 2 977 580. Le contrôle de la taille permet d'apporter des propriétés nouvelles et une meilleure maîtrise de ses propriétés à la fois hydrophiles et hydrophobes, c'est-à-dire amphiphiles.

### Analyse et caractérisation structurelle d'un phyllosilicate synthétique convenant à l'invention

Un phyllosilicate synthétique convenant à l'invention peut être caractérisé par différents paramètres, à savoir des bandes d'absorption en infrarouge, sa taille, sa pureté, comme détaillé ci-après.

Dans certaines conditions, des analyses telles que la résonance magnétique nucléaire en particulier au 29Si peuvent être utile pour la caractérisation d'un phyllosilicate synthétique convenant à l'invention. De même, l'analyse thermogravimétrique (ATG) peut être mise en œuvre pour la caractérisation d'un phyllosilicate synthétique convenant à l'invention. Enfin, la diffraction des rayons X peut également être utilisée dans cette optique.

### Infrarouge

### Méthode utilisée

L'appareil utilisé est un spectromètre Nicolet 6700 FTIR à transformée de Fourier, équipé d'une sphère d'intégration, avec un détecteur InGaA et une séparatrice CaF2 et une résolution de 12 cm⁻¹, plus préférentiellement de 8 cm⁻¹ et encore plus préférentiellement de 4 cm⁻¹. Autrement dit les valeurs des bandes d'absorption données dans cette description sont à considérer comme étant à plus ou moins 6 cm⁻¹ et plus préférentiellement à plus ou moins 4 cm⁻¹ et encore plus préférentiellement à plus ou moins 2 cm⁻¹.

Les enregistrements en proche infrarouge de la région d'élongation située à 7 184 cm⁻¹ ont été décomposés par des Pseudo-Voigts à l'aide du logiciel Fityk (Wojdyr, 2010).

Pour visualiser le spectre d'absorption dans une composition comprenant au moins une partie aqueuse, telle qu'une émulsion, il est recommandé de chauffer cette composition à une température correspondant à une température supérieure ou égale à 100 °C (par exemple 120°C) et inférieure ou égale à 500 °C (par exemple 400 °C) afin d'éliminer la partie eau adsorbée et le cas échéant une partie ou la totalité du (des) composé(s) organique(s) présent(s) dans la composition.

Généralement pour confirmer une bande d'absorption, l'homme du métier procède à des agrandissements de stretching. En particulier, ce dernier peut par exemple faire de tels agrandissements à plus ou moins 200 cm⁻¹ de part et d'autre d'une bande d'absorption suspectée.

Un talc naturel est une espèce minérale composée de silicate de magnésium doublement hydroxylé de formule Mg₃Si₄O₁₀(OH)₂, pouvant contenir des traces de nickel, de fer, d'aluminium, de calcium ou de sodium.

Le talc naturel présente un spectre infrarouge ayant une bande d'absorption typique, fine et intense, de 7 184 cm⁻¹ correspondant à la vibration d'élongation 2v Mg₃OH. Le talc naturel possède généralement des éléments chimiques se substituant au magnésium et au silicium dans la structure cristalline qui imposent l'apparition d'au moins une bande d'absorption supplémentaire, en particulier celle correspondant à la vibration d'élongation de 7156 cm⁻¹ attribuable à 2v Mg₂FeOH.

Le spectre du phyllosilicate synthétique convenant à l'invention se différencie d'un talc naturel par une bande d'absorption de 7 200 cm⁻¹ correspondant à la vibration d'élongation attribuée aux groupes silanols Si-OH en bordure des feuillets.

Pour confirmer cette bande d'absorption, l'homme du métier peut procéder à un agrandissement de stretching et en particulier dans la zone de 7 400 cm⁻¹ - 7 000 cm⁻¹, et plus particulièrement dans la zone de 7 300 cm⁻¹ - 7 100 cm⁻¹.

De préférence, le spectre du phyllosilicate synthétique selon l'invention se caractérise également en ce qu'il ne présente pas de bande d'absorption de 7 156 cm⁻¹, correspondant à la bande d'absorption de Mg₂FeOH.

De préférence, le spectre du phyllosilicate synthétique selon l'invention se caractérise également par la bande d'absorption de 7 184 cm⁻¹ commune au talc naturel.

Il est à noter qu'en présence d'eau adsorbée, par exemple résiduelle, une bande d'absorption large est détectable, facilement identifiable, par exemple de 5 500 cm⁻¹.

En outre, une composition selon la présente invention comprenant ledit phyllosilicate synthétique, présente une bande d'absorption infrarouge à 7 200 cm⁻¹ correspondant à la vibration d'élongation attribuée aux groupes silanols Si-OH en bordure des feuillets.

Avantageusement, la composition selon la présente invention comprenant ledit phyllosilicate synthétique, est caractérisée par une absence de bande d'absorption infrarouge de 7 156 cm⁻¹, correspondant à la bande de vibration de Mg₂FeOH.

La composition selon la présente invention comprenant ledit phyllosilicate synthétique, présente également, de préférence, une bande d'absorption infrarouge à 7 184 cm⁻¹ correspondant à la vibration d'élongation 2v Mg₃OH.

Dans une composition selon l'invention, il est à noter qu'en présence d'eau adsorbée, par exemple résiduelle, une bande d'absorption infrarouge large est détectable, facilement identifiable, par exemple de 5 500 cm⁻¹.

### Taille

### Méthode utilisée

Afin de réaliser l'analyse granulométrique des phyllosilicates synthétiques convenant à l'invention, la spectroscopie de corrélation de photons a été utilisée. Cette technique analytique permet d'accéder à la taille de particules en se basant sur le principe de diffusion dynamique de la lumière. Ce dispositif mesure au cours du temps l'intensité de la lumière diffusée par les particules à un angle θ considéré et les rayons diffusés sont ensuite traités par l'algorithme de Padé-Laplace.

Cette technique, non destructive, nécessite une mise en solution des particules. La mesure granulométrique obtenue par cette technique correspond à la valeur du diamètre hydrodynamique de la particule, c'est-à-dire qu'il comprend à la fois la taille de la particule mais aussi l'épaisseur de la couche d'hydratation.

Les analyses ont été réalisées à l'aide d'un granulomètre VASC0-2 de Cordouan. Dans le but d'obtenir une information statistique quant à la distribution de particules, le logiciel NanoQ™ a été utilisé en mode multi-acquisition avec l'algorithme Padé-Laplace.

Ainsi, un phyllosilicate synthétique convenant à l'invention, sous la forme de gel aqueux ou hydroalcoolique, possède avantageusement une taille moyenne allant de 300 nm à 500 nm.

Ces caractéristiques sont avantageuses vis-à-vis d'un talc naturel dont l'une des contraintes est la dimension non contrôlée de ses particules.

### Pureté

Le phyllosilicate synthétique considéré selon l'invention présente un degré de pureté d'au moins 99,90 %, de préférence d'au moins 99,99 %.

Il est ainsi avantageusement dénué d'impuretés ou de composés indésirables dont font partie notamment des asbestes comme l'amiante (serpentine), la chlorite, les carbonates, les métaux lourds, les sulfures de fer, etc., qui sont généralement associées avec le talc naturel et/ou incorporés dans la structure des talcs naturels.

### RMN (Résonance Magnétique Nucléaire)

### Méthodes utilisées

Les spectres RMN du silicium 29 (²⁹Si) ont été enregistrés sur un spectromètre BRUKER Avance 400 (9,4 T). La référence des déplacements chimiques est le tétraméthylsilane (TMS). Les échantillons ont été placés dans des rotors en zircone de 4 mm. La vitesse de rotation autour de l'angle magique (MAS) a été réglée à 8 kHz. Les expériences ont été effectuées à la température ambiante de 21 °C.

Les spectres ²⁹Si ont été obtenus soit par polarisation directe (rotation de 30°) avec un délai de recyclage de 60 s soit par polarisation croisée (CP) entre le 1H et le 29Si (temps de recyclage de 5 s et temps de contact de 3 ms).

En RMN du silicium (²⁹Si), le talc naturel présente un seul pic à -97 ppm.

En RMN du silicium (²⁹Si), contrairement au talc naturel, le spectre du phyllosilicate synthétique conforme à l'invention laisse apparaître deux pics: l'un situé à -95 ppm et l'autre situé à -97 ppm, et ce sans nécessiter de fractionnement granulométrique à une taille inférieure à 500 nm.

### ATG (analyse thermogravimétrique)

### Méthode utilisée

Les enregistrements ont été réalisés à l'aide d'une thermobalance Perkin Elmer Diamonds.

Pour chaque analyse, environ 20 mg d'échantillon ont été nécessaires. Au cours de l'analyse, l'échantillon est soumis à une montée de température allant de 30°C à 1 200 °C avec un pas de 10 °C.min⁻¹ sous un flux de 100 mL.min⁻¹ d'air.

L'analyse thermogravimétrique d'un phyllosilicate synthétique conforme à l'invention montre une stabilité thermique plus basse (autour de 800 °C) que celle du talc naturel et elle est caractérisée par quatre pertes de masse contrairement au talc naturel qui n'en possède qu'une seule, aux alentours de 900 °C.

Pour établir ces pertes de masses il est utile de se référer à l'article Angela Dumas, François Martin, Christophe Le Roux, Pierre Micoud, Sabine Petit, Eric Ferrage, Jocelyne Brendle, Olivier Grauby, Mike Greenhill-Hooper « Phyllosilicates synthesis: a way of accessing edges contributions in NMR and FTIR spectroscopies. Example of synthetic talc » Phys Chem Minerais, publié le 27 février 2013.

### Diffraction des rayons X

### Méthode utilisée

L'analyse du diffractogramme des rayons X, notamment à l'aide des matériels et méthode utilisés pour les analyses en diffraction des rayons X, sont détaillés dans la demande FR 2 977 580.

De préférence, étant donné que la diffraction aux rayons X ne se fait que sur des solides, pour visualiser le spectre d'absorption dans une composition comprenant au moins une partie aqueuse, telle qu'une émulsion, il est recommandé de chauffer cette composition à une température correspondant à une température supérieure ou égale à 100 °C (par exemple 120 °C) et inférieure ou égale à 500 °C (par exemple 400 °C) afin d'éliminer la partie eau adsorbée et le cas échéant une partie ou la totalité du (des) composé(s) organique(s) présent(s) dans la composition.

Le diffractogramme de rayons X du phyllosilicate synthétique convenant à l'invention présente les mêmes positions des raies de diffraction que celles du talc naturel, à l'exception d'une raie. En effet, le talc naturel présente une raie de diffraction à 9,36 Å tandis que le phyllosilicate synthétique conforme à l'invention présente une raie de diffraction supérieure à 9,4 Å, et pouvant aller jusqu'à 9,8 Å.

Le phyllosilicate synthétique conforme à l'invention présente une raie de diffraction supérieure à 9,4 Å et inférieure ou égale à 9,8 Å.

Le phyllosilicate synthétique conforme à l'invention présente de préférence une raie de diffraction supérieure ou égale à 9,5 Å, avantageusement supérieure ou égale à 9,6 Å, et préférentiellement supérieure ou égale à 9,7 Å.

Le phyllosilicate synthétique conforme à l'invention présente de préférence une raie de diffraction inférieure ou égale à 9,7 Å, avantageusement inférieure ou égale à 9,6 Å, et préférentiellement inférieure ou égale à 9,5 Å.

Le phyllosilicate synthétique conforme à l'invention peut présenter en outre, une raie de diffraction comprise entre 4,60 Å et 4,80 Å, et/ou une raie de diffraction comprise entre 3,10 Å et 3,20 Å et/ou une raie de diffraction comprise entre 1,51 Å et 1,53 Å.

Il est à noter qu'un phyllosilicate synthétique conforme à l'invention est dénué de cations interfoliaires. En effet, cette caractéristique est démontrée par l'absence d'une raie de diffraction des rayons X située à une distance comprise entre 12,00 Å et 18,00 Å, révélant habituellement une phase gonflante présentant des espaces interfoliaires dans lesquels se trouvent des cations interfoliaires et d'éventuelles molécules d'eau.

Il est entendu que, un phyllosilicate synthétique conforme à l'invention étant sous forme de gel, le « % en poids » signifie « % en poids en matière sèche » ou « % en poids en matière active ».

Le phyllosilicate synthétique sous forme gel peut être présent dans une composition selon l'invention en une teneur allant de 0,01 % à 40 % en poids en matière active, en particulier allant de 0,05 % à 30 % en poids en matière active, de préférence allant de 0,1 % à 20 % en poids en matière active, et plus préférentiellement de 0,2 % à 10 % en poids en matière active, par rapport au poids total de la composition.

### INGREDIENTS ADDITIONNELS

Comme indiqué ci-dessus, une composition selon l'invention comprend en outre au moins un ingrédient distinct du phyllosilicate synthétique requis selon l'invention.

Cet ingrédient additionnel peut avantageusement être choisi parmi les composés et additifs conventionnellement considérés pour la formulation des compositions cosmétiques.

Il peut ainsi s'agir d'au moins un ingrédient classiquement retenu pour former une phase grasse ou une phase aqueuse dans une composition.

Ainsi, selon un premier mode de réalisation, une composition selon l'invention comprend à titre d'ingrédient additionnel au moins un composé constitutif d'une phase grasse. Celui-ci peut en particulier être choisi parmi une huile hydrocarbonée ou siliconée, polaire ou apolaire, volatile ou non volatile, une cire, un composé pâteux, et un de leurs mélanges.

Selon un mode de réalisation, une composition selon l'invention comprend à titre d'ingrédient additionnel au moins une huile siliconée volatile ou non volatile.

Selon un mode de réalisation, une composition selon l'invention comprend à titre d'ingrédient additionnel au moins une huile hydrocarbonée, cette huile étant notamment choisie parmi les huiles hydrocarbonées volatiles en C₈-C₁₆, les éthers de synthèse ayant de 10 à 40 atomes de carbone, les esters de synthèse, les esters de polyols et les esters du pentaérythritol, les alcools gras liquides à température ambiante à chaîne carbonée ramifiée et/ou insaturée ayant de 8 à 26 atomes de carbone, les acides gras supérieurs en C₁₂-C₂₂ ; et leurs mélanges.

Selon une variante de réalisation, une composition selon l'invention comprend à titre d'ingrédient additionnel selon l'invention au moins un composé constitutif d'une phase aqueuse, en particulier choisi parmi les alcools, de préférence les monoalcools inférieurs ayant de 1 à 5 atomes de carbone, et les polyols, de préférence choisi parmi les polyols présentant de 2 à 32 atomes de carbone.

Selon une variante de réalisation, une composition selon l'invention comprend à titre d'ingrédient additionnel selon l'invention au moins un alcool gras ou un ester.

Il peut également s'agir d'un ingrédient plus particulièrement considéré pour structurer et/ou stabiliser l'architecture d'une composition cosmétique à l'image par exemple des tensioactifs, gélifiants et charges.

Selon un mode de réalisation, une composition selon l'invention comprend à titre d'ingrédient additionnel selon l'invention au moins un composé choisi parmi les tensioactifs non ioniques, anioniques ou amphotères ; les charges ; les agents gélifiants ; et leurs mélanges.

Il peut également être choisi parmi les ingrédients dédiés à procurer une activité particulière tel que les actifs cosmétiques comme par exemple les agents hydratants, anti-âges et/ou vitamines, les ingrédients ayant vocation à conférer à la composition un effet visuel ou sensoriel spécifique à l'image des matières colorantes de type pigments, nacres et parfums ou encore à lui garantir un défaut de contamination en cours du temps comme les conservateurs.

Selon un mode de réalisation, une composition selon l'invention comprend à titre d'ingrédient additionnel selon l'invention au moins un composé choisi parmi les actifs cosmétiques, et notamment parmi les agents hydratant, anti-âge, blanchissant, agents anti transpirants ; tensioactifs ioniques et non ioniques ; les vitamines ; les filtres anti-UV ; les matières colorantes, de préférence choisies parmi les pigments et les nacres ; et les parfums, de préférence les huiles essentielles.

Selon une variante de réalisation, une composition selon l'invention comprend à titre d'ingrédient additionnel selon l'invention au moins de l'acide hyaluronique, réticulé ou non, ou l'un de ses dérivés ou sels, en particulier l'un de ses sels de cation monovalent choisi parmi le sodium et le potassium, ou multivalent choisi parmi le calcium, le zinc, le cuivre et le manganèse.

Bien entendu, une composition peut contenir un ou plusieurs des ingrédients définis ci-dessus et tels que détaillés ci-après.

### Phase grasse

Une composition selon l'invention peut également comprendre une phase grasse.

Au sens de l'invention, une phase grasse inclut tout corps gras liquide, généralement des huiles (aussi appelée phase grasse liquide ou huileuse), ou solide à l'image des cires ou composés pâteux (aussi appelée phase grasse solide).

La phase grasse d'une composition selon l'invention peut être uniquement constituée d'une phase grasse liquide ou d'une phase grasse solide, ou peut comprendre un mélange d'une phase grasse liquide et d'une phase grasse solide.

Cette phase grasse peut avantageusement aller de 10 % à 90 % en poids, en particulier de 15 % à 60 % en poids, de préférence de 20 % à 40 % en poids, par rapport au poids total de la composition.

Selon un mode de réalisation particulier, une composition conforme à l'invention peut contenir moins de 5 % en poids de phase grasse, voire moins de 2% en poids de phase grasse par rapport au poids total de la composition, voire être exempte de phase grasse.

Ainsi, une composition selon l'invention, et en particulier sa phase grasse, peut contenir au moins un ingrédient choisi parmi les corps gras siliconés tels que les huiles, les gommes et les cires de silicone; les corps gras non siliconés tels que les huiles hydrocarbonées ou fluorées, les pâteux et les cires d'origine végétale, minérale, animale et/ou synthétique; les acides gras ayant de 8 à 32 atomes de carbone; les esters et les éthers de synthèse; les hydrocarbures linéaires ou ramifiés, d'origine minérale ou synthétique, et les alcools gras ayant de 8 à 26 atomes de carbone.

### Huiles

On entend par « huile », tout corps gras sous forme liquide à température ambiante (25 °C) et à pression atmosphérique (760 mmHg).

Une phase huileuse convenant à la préparation des compositions cosmétiques selon l'invention peut comprendre au moins une huile hydrocarbonée ou siliconée, polaire ou apolaire, ou un de leurs mélanges.

Au sens de la présente invention, on entend par « huile siliconée », une huile comprenant au moins un atome de silicium, et notamment au moins un groupe Si-O.

On entend par « huile fluorée », une huile comprenant au moins un atome de fluor.

On entend par « huile hydrocarbonée », une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyles ou acides.

Les huiles peuvent être volatiles ou non volatiles.

Elles peuvent être d'origine végétale, minérale ou synthétique. Selon une variante de réalisation, les huiles d'origine végétale sont préférées.

Au sens de la présente invention, on entend par « huile non volatile », une huile ayant une pression de vapeur inférieure à 0,13 Pa.

Par « huile volatile », on entend, au sens de l'invention, toute huile susceptible de s'évaporer au contact de la peau en moins d'une heure, à température ambiante et pression atmosphérique. L'huile volatile est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1 300 Pa (0,01 à 10 mm de Hg).

### Huiles volatiles

Selon un mode de réalisation, une composition selon l'invention peut notamment comprendre au moins une huile volatile hydrocarbonée ou siliconée.

Une huile volatile hydrocarbonée peut notamment être choisie parmi les huiles volatiles hydrocarbonées ayant de 8 à 16 atomes de carbone, les alcanes ramifiés en C₈-C₁₆ comme les iso-alcanes (appelées aussi isoparaffines) en C₈-C₁₆, l'isododécane, l'isodécane, l'isohexadécane et par exemple les huiles vendues sous les noms commerciaux d'Isopars ou de Permetyls, les esters ramifiés en C₈-C₁₆ comme le néopentanoate d'iso-hexyle, et leurs mélanges.

De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane et l'isohexadécane.

On peut également citer les alcanes linéaires volatils comprenant de 8 à 16 atomes de carbone, en particulier de 10 à 15 atomes de carbone, et plus particulièrement de 11 à 13 atomes de carbone, comme par exemple le n-dodécane (C12) et le n-tétradécane (C14) vendus par Sasol respectivement sous les références PARAFOL 12-97 et PARAFOL 14-97, ainsi que leurs mélanges, le mélange undécane-tridécane, les mélanges de n-undécane (C11) et de n-tridécane (C13) obtenus aux exemples 1 et 2 de la demande WO 2008/155059 de la Société Cognis, et leurs mélanges.

Une huile volatile siliconée peut notamment être choisie parmi les huiles volatiles siliconées linéaires telles que l'hexamethyldisiloxane, l'octamethyltrisiloxane, le decamethyltetrasiloxane, le tetradecamethylhexasiloxane, l'hexadecamethylheptasiloxane et le dodecaméthylpentasiloxane.

Une huile volatile siliconée peut être choisie parmi les huiles volatiles siliconées cycliques, telles que par exemple l'hexamethylcyclotrisiloxane, l'octamethylcylotetrasiloxane, le décamethylcyclopentasiloxane, le cyclohexasiloxane et le dodécaméthylcyclohexasiloxane.

### Huiles non volatiles

Une composition selon l'invention peut également comprendre, à titre d'ingrédient additionnel, au moins une huile non volatile choisie parmi les huiles hydrocarbonées et/ou les huiles siliconées non volatiles.

Comme huile hydrocarbonée non volatile pouvant convenir à l'invention, on peut notamment citer :
- les huiles hydrocarbonées d'origine végétale,
- les éthers de synthèse ayant de 10 à 40 atomes de carbone, comme le dicapryl ether,
- les esters de synthèse, comme les huiles de formule R₁COOR₂, dans laquelle R₁ représente un reste d'un acide gras linéaire ou ramifié comportant de 8 à 40 atomes de carbone et R₂ représente une chaîne hydrocarbonée, notamment, ramifiée contenant de 1 à 40 atomes de carbone à condition que R₁ + R₂ soit ≥ 10.

Ces esters peuvent être notamment choisis parmi les esters d'alcool et d'acide gras, comme par exemple, l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate d'isopropyle, le stéarate d'octyle, les esters hydroxylés, comme le lactate d'isostéaryle, l'hydroxystéarate d'octyle, les ricinoléates d'alcools ou de polyalcools, le laurate d'hexyle, les esters de l'acide néopentanoïque, comme le néopentanoate d'isodécyle, le néopentanoate d'isotridécyle, et les esters de l'acide isononanoïque, comme l'isononanoate d'isononyle, et l'isononanoate d'isotridécyle,
- les esters de polyols et les esters du pentaérythritol, comme le tétrahydroxystéarate/tétraisostéarate de dipentaérythritol,
- les alcools gras liquides à température ambiante à chaîne carbonée ramifiée et/ou insaturée ayant de 8 à 26 atomes de carbone, comme le 2-octyldodécanol, l'alcool isostéarylique, l'alcool oléique, d'alcools gras utilisables dans le cadre de la présente invention, on peut notamment citer l'alcool laurique, myristique, cétylique, stéarylique, isostéarylique, palmitique, oléique, cétéarylique ou cétylstéarylique (mélange d'alcool cétylique et stéarylique), béhénique, érucique, arachidylique, l'alcool 2-hexyldécylique, l'alcool isocétylique, et leurs mélanges
- les acides gras supérieurs en C₁₂-C₂₂, tels que l'acide oléique, l'acide linoléique, l'acide linolénique, et leurs mélanges.

Pour sa part, l'huile siliconée non volatile peut être choisie parmi :
- les huiles siliconés non phénylées, comme par exemple la caprylyl méthycone ou encore les polydiméthylsiloxanes (PDMS), et
- les huiles siliconés phénylées, comme par exemple les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, la triméthylpentaphényltrisiloxane, et leurs mélanges ; ainsi que les mélanges de ces différentes huiles.

Ainsi, selon un mode de réalisation, une composition selon l'invention comprend au moins un ingrédient additionnel, ledit ingrédient additionnel étant :
- au moins une huile hydrocarbonée non volatile choisie parmi les éthers de synthèse ayant de 10 à 40 atomes de carbone, les esters de synthèse, les esters de polyols et les esters du pentaérythritol, les alcools gras liquides à température ambiante à chaîne carbonée ramifiée et/ou insaturée ayant de 8 à 26 atomes de carbone et les acides gras supérieurs en C₁₂-C₂₂ ; et/ou
- au moins une huile siliconée non volatile choisie parmi les huiles siliconés non phénylées et les huiles siliconés phénylées, et/ou
- l'un de leurs mélanges.

De préférence, une composition selon l'invention comprend à titre d'ingrédient additionnel selon l'invention au moins une huile siliconée volatile et/ou non volatile.

Une composition selon l'invention peut également comprendre un ou plusieurs ingrédients additionnels solubilisés dans les huiles tels que des résines de silicone comme la trifluorométhyl-C₁₋₄-alkyldimethicone et la Trifluoropropyldimethicone, et les élastomères de silicone comme les produits commercialisés sous les dénominations « KSG » par la société Shin-Etsu, sous la dénomination « Trefil » par la société Dow Corning ou sous les dénominations « Gransil » par la société Grant Industries ; et leurs mélanges.

La phase huileuse selon l'invention peut en particulier posséder une contrainte seuil supérieure à 1,5 Pa et de préférence supérieure à 10 Pa. Cette valeur de contrainte seuil traduit une texture de type gel de cette phase huileuse.

Une composition selon l'invention peut également comprendre au moins un corps gras solide choisi parmi les gommes, les cires siliconées ou hydrocarbonées, les pâteux et les cires d'origine végétale, minérale et/ou synthétique.

### Cires

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition, en particulier sous forme de stick; elles peuvent être hydrocarbonées, fluorées et/ou siliconées et être d'origine végétale, minérale, et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

### Composé pâteux

Par « pâteux » au sens de la présente invention, on entend désigner un composé gras lipophile, à changement d'état solide/liquide réversible, et comportant à la température de 25 °C une fraction liquide et une fraction solide.

Un composé pâteux est avantageusement choisi parmi :
- la lanoline et ses dérivés,
- les composés fluorés polymères ou non,
- les composés siliconés polymères ou non,
- les polymères vinyliques, notamment:
- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en C₈-C₃₀,
- les oligomères homo et copolymères d'esters vinyliques ayant des groupements alkyles en C₈-C₃₀,
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- le polylaurate de vinyle ; et
- leurs mélanges.

### Phase Aqueuse

Comme il ressort de ce qui précède une composition selon l'invention contient une phase aqueuse formée au moins en partie par le gel aqueux ou hydroalcoolique de phyllosilicate synthétique.

Selon un mode de réalisation, le gel aqueux ou hydroalcoolique de phyllosilicate synthétique selon l'invention constitue la phase aqueuse, c'est-à-dire que la phase aqueuse est exclusivement constituée de ce gel.

Avantageusement, une composition selon l'invention, contient en outre au moins un ingrédient annexe hydrophile.

Ainsi, la phase aqueuse d'une composition selon l'invention peut en outre comprendre de l'eau et/ou un solvant hydrosoluble.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans la composition de l'invention peuvent en outre être volatils.

Ainsi, une composition selon l'invention peut comprendre au moins un solvant hydrosoluble choisi parmi les monoalcools inférieurs ayant de 1 à 5 atomes de carbone tels que l'éthanol et l'isopropanol et les polyols.

Les polyols convenant avantageusement pour la formulation d'une composition selon la présente invention sont ceux présentant notamment de 2 à 32 atomes de carbone, de préférence 3 à 16 atomes de carbone.

Avantageusement, le polyol peut être par exemple choisi parmi l'éthylèneglycol, le pentaérythritol, le triméthylolpropane, le propylène glycol, le 1,3 propanediol, le butylène glycol, l'isoprène glycol, le pentylène glycol, l'héxylène glycol, le glycérol, les polyglycérols, tels que les oligomères du glycérol comme le diglycérol, les polyéthylènes glycols, et leurs mélanges.

Selon un mode de réalisation préféré de l'invention, ledit polyol est choisi parmi l'éthylèneglycol, le pentaérythritol, le triméthylolpropane, le propylène glycol, le butylène glycol, le glycérol, les polyglycérols, les polyéthylènes glycols et leurs mélanges.

Selon un mode particulier, la composition de l'invention peut comprendre au moins du butylène glycol et/ou du glycérol.

La phase aqueuse peut être présente dans la composition en une teneur allant de 5 % à 98 %, 5 % à 95 %, mieux de 30 % à 80 % en poids, de préférence de 40 % à 75 % en poids, par rapport au poids total de ladite composition.

### Ingrédients choisis parmi les tensioactifs. les gélifiants et les charges

### Tensioactifs

Une composition selon l'invention peut comprendre de 0,1 % à 10 % en poids de tensioactifs(s), de préférence de 0,5 % à 5 % en poids, par rapport au poids total de la composition et en particulier de 1,0 % à 4 % en poids, par rapport au poids total de la composition.

Les agents tensioactifs peuvent être choisis parmi des agents tensioactifs non ioniques, anioniques, amphotères et leurs mélanges. On peut se reporter au document « Encyclopedia of Chemical Technology, KIRK-OTHMER », volume 22, pp. 333-432, 3ème édition, 1979, WILEY, pour la définition des propriétés et des fonctions émulsionnantes des agents tensioactifs, en particulier pp. 347-377 de cette référence, pour les agents tensioactifs anioniques, amphotères et non ioniques.

Une composition selon l'invention peut comprendre au moins un tensioactif hydrocarboné, un tensioactif siliconé, et leur mélange.

Des exemples de tensioactifs hydrocarbonés convenant à l'invention sont décrits ci-après.

### Tensioactifs non ioniques

Les tensioactifs non ioniques peuvent être choisis notamment parmi les alkyl- et polyalkyl- esters de poly(oxyde d'éthylène), les alcools oxyalkylénés, les alkyl- et polyalkyl- éthers de poly(oxyde d'éthylène), les alkyl- et polyalkyl- esters de sorbitan, polyoxyéthylénés ou non, les alkyl- et polyalkyl- éthers de sorbitan, polyoxyéthylénés ou non, les alkyl- et polyalkyl- glycosides ou polyglycosides, en particulier les alkyl- et polyalkyl-glucosides ou polyglucosides, les alkyl- et polyalkyl- esters de sucrose, les alkyl- et polyalkyl-esters de glycérol, polyoxyéthylénés ou non, les alkyl- et polyalkyl- éthers de glycérol, polyoxyéthylénés ou non et leurs mélanges.
1) Comme alkyl- et polyalkyl- esters de poly(oxyde d'éthylène), on utilise de préférence ceux ayant un nombre de motifs d'oxyde d'éthylène (OE) allant de 2 à 200. On peut par exemple citer le stéarate 40 OE, le stéarate 50 OE, le stéarate 100 OE, le laurate 20 OE, le laurate 40 OE, le distéarate 150 OE.
2) Comme alkyl- et polyalkyl- éthers de poly(oxyde d'éthylène), on utilise de préférence ceux ayant un nombre de motifs d'oxyde d'éthylène (OE) allant de 2 à 200. On peut par exemple citer le cétyl éther 23 OE, l'oléyl éther 50 OE, le phytostérol 30 OE, le stéareth 40, le stéareth 100, le béhéneth 100.
3) Comme alcools oxyalkylénés, en particulier oxyéthylénés et/ou oxypropylénés on utilise de préférence ceux pouvant comporter de 1 à 150 motifs oxyéthylène et/ou oxypropylène, en particulier ayant de 20 à 100 motifs oxyéthylène, en particulier les alcools gras, notamment en C₈-C₂₄, et de préférence en C₁₂-C₁₈, éthoxylés tels que l'alcool stéarylique éthoxylé à 20 motifs oxyéthylène (nom CTFA « Steareth-20 ») comme le BRIJ 78 commercialisé par la société UNIQEMA, l'alcool cétéarylique éthoxylé à 30 motifs oxyéthylène (nom CTFA « Ceteareth-30 ») et le mélange d'alcools gras en C₁₂-C₁₅ comportant 7 motifs oxyéthylène (nom CTFA « C₁₂₋₁₅ Pareth-7 ») comme celui commercialisé sous la dénomination NEODOL 25-7® par SHELL CHEMICALS ; ou en particulier les alcools oxyalkylénés (oxyéthylénés et/ou oxypropylénés) ayant de 1 à 15 motifs oxyéthylène et/ou oxypropylène, en particulier les alcools gras en C₈-C₂₄, et de préférence en C₁₂-C₁₈, éthoxylés tels que l'alcool stéarylique éthoxylé à 2 motifs oxyéthylène (nom CTFA « Steareth-2 ») tel que le BRIJ 72 commercialisé par la société UNIQEMA ;
4) Comme alkyl- et polyalkyl- esters de sorbitan, polyoxyéthylénés ou non, on utilise de préférence ceux ayant un nombre de motifs d'oxyde d'éthylène (OE) allant de 0 à 100. On peut par exemple citer le laurate de sorbitan 4 ou 20 OE, en particulier le polysorbate 20 (ou polyoxyéthylène (20) sorbitan monolaurate) tel que le produit Tween 20 commercialisé par la société Uniqema, le palmitate de sorbitan 20 OE, le stéarate de sorbitan 20 OE, l'oléate de sorbitan 20 OE ou encore les Crémophor (RH 40, RH 60 ...) de chez BASF.
5) Comme alkyl- et polyalkyl- éthers de sorbitan, polyoxyéthylénés ou non, on utilise de préférence ceux ayant un nombre de motifs d'oxyde d'éthylène (OE) allant de 0 à 100.
6) Comme alkyl- et polyalkyl- glucosides ou polyglucosides, on utilise de préférence ceux contenant un groupe alkyle comportant de 6 à 30 atomes de carbone et de préférence de 6 à 18, voire de de 8 à 16 atomes de carbone, et contenant un groupe glucoside comprenant de préférence de 1 à 5, notamment 1, 2 à 3 unités de glucoside. Les alkylpolyglucosides peuvent être choisis par exemple parmi le décylglucoside (Alkyl-C₉/C₁₁-polyglucoside (1.4)) comme le produit commercialisé sous la dénomination Mydol 10® par la société Kao Chemicals ou le produit commercialisé sous la dénomination Plantacare 2000 UP® par la société Henkel et le produit commercialisé sous la dénomination ORAMIX NS 10® par la société SEPPIC ; le caprylyl/capryl glucoside comme le produit commercialisé sous la dénomination Plantacare KE 3711® par la société Cognis ou ORAMIX CG 110® par la société SEPPIC ; le laurylglucoside comme le produit commercialisé sous la dénomination Plantacare 1200 UP® par la société Henkel ou Plantaren 1200 N® par la société Henkel ; le cocoglucoside comme le produit commercialisé sous la dénomination Plantacare 818 UP® par la société Henkel ; le caprylylglucoside comme le produit commercialisé sous la dénomination Plantacare 810 UP® par la société Cognis, ou encore des tensioactifs du types alkylpolyglucoside vendus sous la référence commerciale Montanov ; et leurs mélanges.

Plus généralement, les tensioactifs de type alkylpolyglycoside sont définis plus spécifiquement par la suite.
7) Comme alkyl- et polyalkyl- esters de sucrose, on peut citer par exemple les Crodesta F150, le monolaurate de saccharose commercialisé sous la dénomination Crodesta SL 40, les produits commercialisés par Ryoto Sugar Ester comme par exemple, le palmite de sucrose commercialisé sous la référence le Ryoto Sugar Ester P1670, le Ryoto Sugar Ester LWA 1695, le Ryoto Sugar Ester 01570.
8) Comme alkyl- et polyalkyl- esters de glycérol, polyoxyéthylénés ou non, on utilise de préférence ceux ayant un nombre de motifs d'oxyde d'éthylène (OE) allant de 0 à 100 et un nombre de motifs glycérol allant de 1 à 30. On peut par exemple citer l'hexaglycéryl monolaurate et le PEG-30 glycéryl stéarate.
9) Comme alkyl- et polyalkyl- éthers de glycérol, polyoxyéthylénés ou non, on utilise de préférence ceux ayant un nombre de motifs d'oxyde d'éthylène (OE) allant de 0 à 100 et un nombre de motifs glycérol allant de 1 à 30. A titre d'exemple, on peut citer Nikkol Batyl alcohol 100, Nikkol chimyl alcohol 100.

A titre de tensioactif non ionique selon l'invention peut également être cité un mélange mono/distéarate de glycéryle / stéarate de polyéthylène glycol (100 OE).

### Tensioactifs anioniques

Les tensioactifs anioniques peuvent être choisis parmi les alkyl éther sulfates, les carboxylates, les dérivés des amino acides, les sulfonates, les iséthionates, les taurates, les sulfosuccinates, les alkylsulfoacétates, les phosphates et alkylphosphates, les polypeptides, les sels métalliques d'acides gras en C₁₀-C₃₀, notamment en C₁₂-C₂₀, en particulier les stéarates métalliques et leurs mélanges.
1) Comme alkyl éther sulfates, on peut citer par exemple le lauryl éther sulfate de sodium (C12-14 70-30) (2,2 OE) commercialisé sous les dénominations SIPON AOS225 ou TEXAPON N702 par la société Henkel, le lauryl éther sulfate d'ammonium (C12-14 70-30) (3 OE) commercialisé sous la dénomination SIPON LEA 370 par la société Henkel, l'alkyl (C₁₂-C₁₄) éther (9 OE) sulfate d'ammonium commercialisé sous la dénomination RHODAPEX AB/20 par la société Rhodia Chimie, et le mélange de lauryl et oléyl éther sulfate de sodium et de magnésium commercialisé sous la dénomination EMPICOL BSD 52 par la société Albright & Wilson.
2) Comme carboxylates, on peut citer par exemple les sels (par exemple alcalins) de N-acylaminoacides, les glycolcarboxylates, les amido éthercarboxylates (AEC) et les sels d'acides carboxyliques polyoxyéthylénés.

Le tensioactif du type glycol carboxylate peut être choisi parmi les alkyl glycol carboxyliques ou 2-(2-hydroxyalkyloxy acétate), leurs sels et leurs mélanges. Ces alkyl glycol carboxyliques comportent une chaîne alkyle linéaire ou ramifiée, saturée ou insaturée, aliphatique et/ou aromatique, ayant de 8 à 18 atomes de carbone. Ces carboxyliques peuvent être neutralisés par des bases minérales telles que la potasse ou la soude.

Comme tensioactifs du type glycol carboxyliques, on peut citer par exemple le lauryl glycol carboxylate de sodium ou 2-(2-hydroxyalkyloxy acétate de sodium) tel que le produit commercialisé sous la dénomination Beaulight Shaa® par la société Sanyo, Beaulight LCA-25N® ou la forme acide correspondante Beaulight Shaa (Acid form)®.

Comme amido éthercarboxylate (AEC), on peut citer par exemple le lauryl amido éther carboxylate de sodium (3 OE), commercialisé sous la dénomination AKYPO FOAM 30® par la société Kao Chemicals.

Comme sel d'acide carboxylique polyoxyéthyléné, on peut citer par exemple le lauryl éther carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX® par la société Nikkol.
3) Comme dérivés des aminoacides, on peut citer notamment les sels alcalins d'aminoacides, tels que :
   - les sarcosinates, comme le lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN® par la société Nikkol.
   - les alaninates, comme le N-lauroyl-N méthyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-méthyl alanine triéthanolamine commercialisé sous la dénomination ALANONE ALTA® par la société Kawaken.
   - les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
      Les sels et/ou dérivés d'acide glutamique sont décrits plus précisément par la suite.
   - les aspartates, comme le mélange de N-lauroyl aspartate de triethanolamine/N-myristoyl aspartate de triéthanolamine commercialisé sous la dénomination ASPARACK® par la société Mitsubishi.
   - les dérivés de glycine (glycinates), comme le N-cocoyl glycinate de sodium commercialisé sous les dénominations AMILITE GCS-12® et AMILITE GCK 12 par la société Ajinomoto.
   - les citrates tels que le mono-ester citrique d'alcools de coco oxyéthylénés (9 moles), commercialisé sous la dénomination WITCONOL EC 1129 par la société Goldschmidt.
   - les galacturonates tels que le dodécyl D-galactoside uronate de sodium commercialisé par la société Soliance.
4) Comme sulfonates, on peut citer par exemple les alpha-oléfines sulfonates comme l'alpha-oléfine sulfonate de sodium (C₁₄₋₁₆) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ;
5) Comme iséthionates, on peut citer les acyliséthionates comme le cocoyl-iséthionate de sodium, tel que le produit commercialisé sous la dénomination JORDAPON CI P® par la société Jordan.
6) Comme taurates, on peut citer le sel de sodium de méthyltaurate d'huile de palmiste commercialisé sous la dénomination HOSTAPON CT PATE® par la société Clariant; les N-acyl N-méthyltaurates comme le N-cocoyl N-méthyltaurate de sodium commercialisé sous la dénomination HOSTAPON LT-SF® par la société Clariant ou commercialisé sous la dénomination NIKKOL CMT-30-T® par la société Nikkol, le palmitoyl méthyltaurate de sodium commercialisé sous la dénomination NIKKOL PMT® par la société Nikkol.
7) Comme sulfosuccinates, on peut citer par exemple le mono-sulfosuccinate d'alcool laurylique (C₁₂/C₁₄ 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C₁₂-C₁₄, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Henkel, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.
8) Comme alkyl sulfoacétate, on peut citer par exemple le mélange de lauryl sulfoacétate de sodium, lauryl éther sulfosuccinate di-sodique, commercialisé sous la dénomination STEPAN-MILD LSB par la société Stepan.
9) Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C₁₂-C₁₃) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie, et le cétylphosphate de potassium commercialisé sous la dénomination ARLATONE MAP 160K par la société Uniqema.
10) Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl aminoacides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic.
11) Comme sels métalliques d'acides gras en C₁₀-C₃₀, notamment en C₁₂-C₂₀, on peut citer en particulier les stéarates métalliques, tels que le stéarate de sodium et le stéarate de potassium, ainsi que les polyhydroxy stéarates.

### Charges

Les compositions conformes à l'invention peuvent également comprendre au moins une charge distincte du phyllosilicate synthétique. Bien entendu, son choix en termes de nature chimique et quantité sera ajusté pour que ne soit pas affecté les propriétés conférées par le phyllosilicate synthétique requis parallèlement selon l'invention.

Les charges peuvent être choisies parmi celles bien connues de l'homme du métier et couramment utilisées dans les compositions cosmétiques. Les charges peuvent être minérales ou organiques, lamellaires ou sphériques.

On peut citer le talc, le mica, la silice hydrophiles ou hydrophobées, les aérogels, la perlite, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

Les charges différentes du phyllosilicate synthétique selon l'invention peuvent représenter de 0,001 % à 15 %, en particulier de 0,5 à 10 % en poids par rapport au poids total de la composition.

### Gélifiants

Un gélifiant selon la présente invention peut être choisi parmi au moins un gélifiant hydrophile, un gélifiant lipophile, et leurs mélanges.

L'agent gélifiant hydrophile peut être choisi parmi les montmorillonites, les hectorites, les bentonites, la beidellite, les saponites, les laponites, les silices pyrogénées, les gélifiants polymériques synthétiques, les gélifiants polymériques naturels ou d'origine naturelle comme par exemple les polysaccharides non amylacés, les amidons natifs, les amidons modifiés, les amidons greffés par un polymère acrylique, les amidons hydrolysés greffés par un polymère acrylique, les polymères à base d'amidon, de gomme et de dérivé cellulosique et leurs mélanges.

Plus particulièrement, un polymère gélifiant hydrophile peut être choisi parmi :
- les homo- ou copolymères d'acides acrylique ou méthacrylique ou leurs sels et leurs esters et en particulier les produits vendus sous les dénominations « VERSICOL F » ou « VERSICOL K » par la société ALLIED COLLOID, « UTRAHOLD 8 » par la société CIBA-GEIGY, les acides polyacryliques de type SYNTHALEN K, et les sels, notamment de sodium, d'acide polyacryliques (répondant au nom INCI sodium acrylate copolymer) et plus particulièrement un polyacrylate de sodium réticulé (répondant au nom INCI sodium acrylate copolymer (and) caprylic/capric triglycéride) vendu sous la dénomination « LUVIGEL EM » par la société,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN N°7 » par la société VANDERBILT, les sels de sodium d'acides polyhydroxycarboxyliques vendus sous la dénomination « HYDAGEN F » par la société HENKEL,
- les copolymères acide polyacryliques/acrylates d'alkyle, de préférence les polymères carboxyvinyliques modifiés ou non, on préfère tout particulièrement selon la présente invention, les copolymères acrylate/C₁₀-C₃₀-alkylacrylate (nom INCI Acrylates/C10-30 Alkyl acrylate Crosspolymer) tels que les produits commercialisés par la société Lubrizol sous les dénominations commerciales PEMULEN TR1, PEMULEN TR2, CARBOPOL 1382, CARBOPOL EDT 2020 et encore plus préférentiellement le PEMULEN TR-2 ;
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL ou SIMULGEL commercialisés par la société SEPPIC,
- les copolymères AMPS/méthacrylates d'alkyle polyoxyéthylénés (réticulé ou non) de type ARISTOFLEX HMS commercialisés par la société CLARIANT,
- les polymères de chitine ou de chitosane anioniques, cationiques, amphotères ou non-ioniques ;
- les polymères de cellulose, distincts de l'alkylcellulose,choisis parmi l'hydroxyéthylcellulose, l'hydroxypropylcellulose, l'hydroxyméthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères associatifs anioniques, cationiques, non-ioniques ou amphotères tels que par exemple les terpolymères d'anhydride maléique/α-oléfine en C₃₀-C₃₈/ maléate d'alkyle, terpolymère acide méthacrylique / acrylate d'éthyle / méthacrylate de stéaryle oxyalkyléné (20 motifs OE) ou terpolymère d'acide méthacrylique/acrylate d'éthyle/méthacrylate de béhényle oxyéthylène (250E), les celluloses modifiées par des groupements comportant au moins une chaîne grasse ou par des groupes polyalkylène glycol éther d'alkyl phénol, les copolymères de vinyl pyrrolidone et de monomères hydrophobes à chaîne grasse ; les copolymères de méthacrylates ou d'acrylates d'alkyles en C₁-C₆ et de monomères amphiphiles comportant au moins une chaîne grasse, les copolymères de méthacrylates ou d'acrylates hydrophiles et de monomères hydrophobes comportant au moins une chaîne grasse, les polyuréthanes associatifs, les copolymères acide acrylique/chlorure d'acrylamidopropyl triméthyl ammonium/ méthacrylate de stéaryle ;
- les polymères d'origine naturelle, éventuellement modifiés, tels que :
   les galactomannanes et leurs dérivés, tels que la gomme de Konjac, la gomme de Gellane, la gomme de Caroube, la gomme de Fennugrec, la gomme de Karaya, la gomme de Tragacanth, la gomme arabique, la gomme d'acacia, la gomme de guar, l'hydroxypropylguar, l'hydroxypropylguar modifié par des groupements méthylcarboxylate de sodium (Jaguar XC97-1, Rhodia), le chlorure de guar hydroxypropyl tri-méthyl ammonium, les dérivés du xanthane, tel que la gomme de xanthane ;
- les alginates et les carraghénanes ;
- les glycoaminoglycanes ;
- l'acide désoxyribonucléique ;
- les muccopolysaccharides tels que l'acide hyaluronique et ses dérivés, en particulier le hyaluronate de sodium, les chondroïtines sulfate, et leurs mélanges.

Selon un mode de réalisation, un gélifiant hydrophile est choisi parmi les silices pyrogénées, les gélifiants polymériques synthétiques, les gélifiants polymériques naturels ou d'origine naturelle, les homo- ou copolymères d'acides acrylique ou méthacrylique, leurs sels ou leurs esters, les copolymères acide polyacryliques/acrylates d'alkyle, les copolymères AMPS/méthacrylates d'alkyle polyoxyéthylénés, réticulés ou non réticulés, les polymères de chitine ou de chitosane anioniques, cationiques, amphotères ou non-ioniques, les polymères de cellulose, distincts de l'alkylcellulose, choisis parmi l'hydroxyéthylcellulose, l'hydroxypropylcellulose, l'hydroxyméthylcellulose, l'éthylhydroxyéthylcellulose, la carboxyméthylcellulose, ainsi que les dérivés quaternisés de la cellulose, les polymères vinyliques et les polymères associatifs anioniques, cationiques, non-ioniques ou amphotères, les polymères d'origine naturelle modifiés ou non modifiés, les alginates et les carraghénanes, les glycoaminoglycanes, l'acide désoxyribonucléique, les muccopolysaccharides en particulier l'acide hyaluronique et ses dérivés, les chondroïtines sulfate, et leurs mélanges.

Selon un mode de réalisation, un phyllosilicate synthétique selon l'invention mis en œuvre en combinaison avec au moins un acide hyaluronique, ou l'un de ses dérivés, notamment l'un de ses sels.

Ce mode de réalisation est notamment illustré dans l'exemple 6 de la présente demande.

L'acide hyaluronique est un glycosaminoglycane linéaire non sulfaté composé d'unités répétitives de D-acide glucuronique et de N-acétyl-D-glucosamine.

Selon l'invention, l'acide hyaluronique ou l'un de ses dérivés possède de préférence un poids moléculaire moyen en nombre allant de 500 Da à 10 MDa, et plus particulièrement allant de 2 kDa à 2 MDa.

Comme acide hyaluronique convenant à la présente invention, on peut notamment citer les acides hyaluroniques d'origine animale, ou obtenus par biotechnologie. Ils sont linéaires ou réticulés, tels que ceux vendus sous la dénomination Hylaform® par la société Genzyme, ou encore les acides hyaluroniques d'origine génétique dont ceux destinés aux rides uperficielles, périorbitaires ou péribuccales, tels que ceux vendus sous la dénomination Restylane Fine Lines® par le Laboratoire Q-Med, ou destinés aux rides profondes, aux dépressions labiomentonnières et ovales du visage, tels que ceux vendus sous les dénominations Perlane® et Restylane Sub-Q® par le Laboratoire Q-Med.

De préférence, comme acide hyaluronique convenant à la présente invention, on peut citer ceux vendus sous la dénomination Restylane® par le Laboratoire Q-Med et sous la dénomination Surgiderm® par le Laboratoire Cornéal.

Peut aussi être avantageusement considéré dans le cadre de l'invention le hyaluronate de sodium :
- de poids moléculaire compris entre 20 et 50 kg/mol commercialisé par la société Soliance sous la dénomination Renovyal LO®,
- de poids moléculaire compris entre 100 et 300 kg/mol commercialisé par la société Soliance sous la dénomination Primalhyal 300®,
- de poids moléculaire compris entre 500 et 1200 kg/mol commercialisé par la société Soliance sous la dénomination Cristalhyal LO®,
- de poids moléculaire compris entre 10 et 200 kg/mol commercialisé par la société Bioland sous la dénomination Bioha®,
- de poids moléculaire compris entre 30 et 60 kg/mol commercialisé par la société Evonik Goldschmidt sous la dénomination Hyacare 50®,
- de poids moléculaire compris entre 100 et 140 kg/mol commercialisé par la société Contripro sous la dénomination Hyactive 120®.

Parmi les sels d'acide hyaluronique, on peut notamment citer les sels de sodium, les sels de potassium, les sels de zinc, les sels d'argent, et leurs mélanges.

Plus particulièrement, comme sels d'acide hyaluronique, on peut citer le hyaluronate de potassium et le hyaluronate de sodium, de préférence le hyaluronate de sodium.

Selon un autre mode de réalisation particulier, une composition peut comprendre au moins un gélifiant lipophile.

Un gélifiant lipophile peut être choisi parmi les gélifiants particulaires, et notamment les argiles modifiées, les silices comme les silices pyrogénées et les aérogels de silice hydrophobe ; les élastomères d'organopolysiloxane ; les polymères semi-cristallins ; les copolymères séquencés hydrocarbonés ; les esters de dextrine ; les polymères à liaison hydrogène ; et leurs mélanges.

Selon un mode de réalisation de l'invention, un gélifiant lipophile est choisi parmi les gélifiants particulaires, les élastomères d'organopolysiloxane, les polymères semi-cristallins, les copolymères séquencés hydrocarbonés, les esters de dextrine, les polymères à liaison hydrogène et leurs mélanges.

A titre représentatif des gélifiants particulaires lipophiles convenant à l'invention peuvent être tout particulièrement cités, les argiles modifiées, les silices comme les silices pyrogénées et les aérogels de silice hydrophobes.

a) Les argiles peuvent être naturelles ou synthétiques et elles sont rendues lipophiles par un traitement avec un sel d'alkyl ammonium comme un chlorure d'ammonium en C₁₀ à C₂₂, par exemple le chlorure de di-stéaryl di-méthyl ammonium.

Elles peuvent être choisies parmi les bentonites en particulier les hectorites et les montmorillonites, les beidellites, les saponites, les nontronites, les sépiolites, les biotites, les attapulgites, les vermiculites et les zéolites.

De préférence, elles sont choisies parmi les hectorites.

De préférence, on utilise à titre d'argiles lipophiles les hectorites modifiées par un chlorure d'ammonium en C₁₀ à C₂₂, comme l'hectorite modifiée par du chlorure de di-stéaryl di-méthyl ammonium telle que, par exemple, celle commercialisée sous la dénomination de Bentone 38V® par la société Elementis ou le gel de bentone dans isododécane commercialisé sous la dénomination Bentone Gel ISD V® (Isododécane 87 %/Disteardimonium Hectorite 10 %/Propylène carbonate 3 %) par la société Elementis.

De l'argile lipophile peut notamment être présente en une teneur allant de 0,1 % à 15 % en poids, en particulier de 0,50.1 % à 10 %, plus particulièrement de 0.2 % à 8 % en poids par rapport au poids total de la composition.
b) la silice pyrogénée est avantageusement traitée hydrophobe en surface. Les groupements hydrophobes peuvent être :
   - des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société Degussa, CAB-O-SIL TS-530® par la société Cabot.
   - des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société Degussa, CAB-O-SIL TS-610® et CAB-O-SIL TS-720® par la société Cabot.
c) Les aérogels de silice sont des matériaux poreux obtenus en remplaçant (par séchage) la composante liquide d'un gel de silice par de l'air.

Les aérogels utilisés selon la présente invention sont plus particulièrement des aérogels de silice hydrophobe, de préférence de silice silylée (nom INCI : silica silylate). Par « silice hydrophobe », on entend toute silice dont la surface est traitée par des agents de silylation, par exemple par des silanes halogénés tels que des alkylchlorosilanes, des siloxanes, en particulier des dimethylsiloxanes tel que l'hexamethyldisiloxane, ou des silazanes, de manière à fonctionnaliser les groupements OH par des groupements silyles Si-Rn, par exemple des groupements triméthylsilyles.

Concernant la préparation de particules d'aérogels de silice hydrophobe modifiés en surface par silylation, on peut se référer au document US 7,470,725.

On utilisera de préférence des particules d'aérogels de silice hydrophobe modifiée en surface par groupements triméthylsilyles, de préférence de nom INCI Silica silylate.

A titre de d'aérogels de silice hydrophobe utilisables dans l'invention, on peut citer par exemple l'aérogel commercialisé sous la dénomination VM-2260 ou VM-2270 (nom INCI : Silica silylate), par la société Dow Corning, dont les particules présentent une taille moyenne d'environ 1000 microns et une surface spécifique par unité de masse allant de 600 à 800 m²/g.

On peut également citer les aérogels commercialisés par la société Cabot sous les références Aerogel TLD 201, Aerogel OGD 201, Aerogel TLD 203, ENOVA® Aerogel MT 1100, ENOVA Aerogel MT 1200.

A titre représentatif des gélifiants polymériques lipophiles convenant à l'invention peuvent être tout particulièrement cités,
a) Les copolymères séquencés hydrocarbonés de l'invention, également appelés copolymères bloc, sont de préférence solubles ou dispersibles dans la phase huileuse.

De tels copolymères blocs hydrocarbonés sont décrits dans la demande US-A-2002/005562 et dans le brevet US-A-5 221 534.

Avantageusement, le copolymère bloc hydrocarboné est un copolymère bloc amorphe de styrène et d'oléfine.

On préfère notamment les copolymères séquencés comprenant au moins un bloc styrène et au moins un bloc comprenant des motifs choisis parmi le butadiène, l'éthylène, le propylène, le butylène, l'isoprène ou un de leurs mélanges.

Selon un mode préféré de réalisation, le copolymère bloc hydrocarboné est hydrogéné pour réduire les insaturations éthyléniques résiduelles après la polymérisation des monomères.

En particulier, le copolymère bloc hydrocarboné est un copolymère, éventuellement hydrogéné, à blocs styrène et à blocs éthylène/alkylène en C₃-C₄.

Selon un mode de réalisation préféré, la composition selon l'invention comprend au moins un copolymère dibloc, de préférence hydrogéné, de préférence choisi parmi les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701E par la société Kraton Polymers.

Selon un autre mode de réalisation préféré, la composition selon l'invention comprend au moins un copolymère tribloc, de préférence hydrogéné, de préférence choisi parmi les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® D1101, Kraton® D1102, Kraton® D1160 par la société Kraton Polymers.

Selon un mode de réalisation de la présente invention, le copolymère bloc hydrocarboné est un copolymère tribloc styrène-éthylène/butylène-styrène.

Selon un mode préféré de réalisation de l'invention, on peut notamment utiliser un mélange d'un copolymère tribloc styrène-butylène/éthylène-styrène et d'un copolymère dibloc styrène-éthylène/butylène, notamment ceux vendus sous la dénomination Kraton® G1657M par la société Kraton Polymers.

Selon un autre mode de réalisation préféré, la composition selon l'invention comprend un mélange de copolymère hydrogéné tribloc styrène-butylène/éthylène-styrène et de polymère étoile hydrogéné éthylène-propylène-styrène, un tel mélange pouvant notamment être dans l'isododécane ou dans une autre huile. De tels mélanges sont par exemple vendus par la société PENRECO sous les dénominations commerciales VERSAGEL® M5960 et VERSAGEL® M5670.

Le(s) copolymère(s) bloc hydrocarboné(s) peu(ven)t être présent en une teneur allant de 0,5 % à 15 % en poids, par rapport au poids total de la composition, de préférence allant de 1 % à 10 % en poids, et encore plus avantageusement de 2 à 8 % en poids par rapport au poids total de la composition.

b) Les polymères à liaison hydrogène convenant à l'invention peuvent être tout particulièrement les polyamides et en particulier les polyamides hydrocarbonés et les polyamides siliconés.

Selon un mode particulier, le polyamide utilisé est un polyamide à terminaison amide de formule (Ia) : 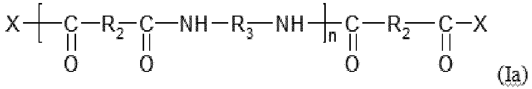 dans laquelle X représente un groupe -N(R₁)₂ dans lequel R₁ est un radical alkyl linéaire ou ramifié en C₈ à C₂₂, pouvant être identique ou différents l'un de l'autre, R₂ est un résidu de dimère diacide en C₂₈-C₄₂, R₃ est un radical éthylène diamine, n'est compris entre 2 et 5 ;
et leurs mélanges.

La phase huileuse d'une composition selon l'invention peut comprendre en outre, de façon additionnelle dans ce cas, au moins un polyamide additionnel de formule (Ib) : 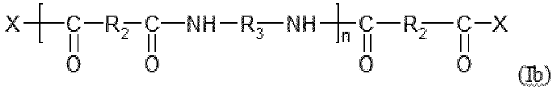 dans laquelle X représente un groupe -OR₁ dans lequel R₁ est un radical alkyl linéaire ou ramifié en C₈ à C₂₂, de préférence en C₁₆ à C₂₂, pouvant être identique ou différents l'un de l'autre, R₂ est un résidu de dimère diacide en C₂₈-C₄₂, R₃ est un radical éthylène diamine, n'est compris entre 2 et 5, tels que les produits commerciaux vendus par la société Arizona Chemical sous les noms Uniclear 80 et Uniclear 100 ou encore Uniclear 80 V, Uniclear 100 V et Uniclear 100 VG, dont le nom INCI est « éthylènediamine/stéaryl dimère dilinoléate copolymère ».

Les polyamides siliconés peuvent préférentiellement être des polymères comprenant au moins un motif de formule (III) ou (IV) : 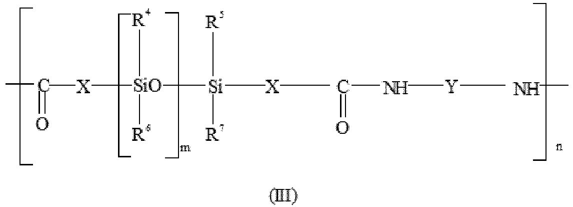 ou 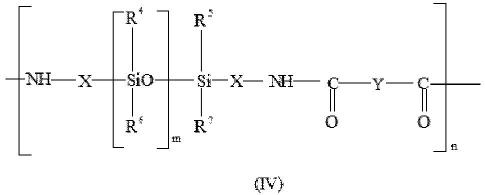 dans lesquelles :
- R⁴, R⁵, R⁶ et R⁷, identiques ou différents, représentent un groupe choisi parmi :
   - les groupes hydrocarbonés, linéaires, ramifiés ou cycliques, en Ci à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
- les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
- Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou Y représente un groupe répondant à la formule : 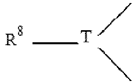 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
- n est un nombre entier allant de 2 à 500, de préférence de 2 à 200 et m est un nombre entier allant de 1 à 1 000, de préférence de 1 à 700 et mieux encore de 6 à 200.

A titre d'exemple de polymère siliconé utilisable, on peut citer un des polyamides siliconés, obtenus conformément aux exemples 1 à 3 du document US 5,981,680.

On peut citer les composés commercialisés par la société Dow Corning sous le nom DC 2-8179 (DP 100) et DC 2-8178 (DP 15) dont le nom INCI est «Nylon-611/dimethicone copolymères» c'est-à-dire des copolymères Nylon-611/dimethicone. Les polymères et/ou copolymères siliconés ont avantageusement une température de transition de l'état solide à l'état liquide allant de 45 °C à 190 °C. De préférence, ils présentent une température de transition de l'état solide à l'état liquide allant de 70 °C à 130 °C et mieux de 80 °C à 105 °C.

c) Par « élastomère d'organopolysiloxane » ou « élastomère de silicone », on entend un organopolysiloxane souple, déformable ayant des propriétés viscoélastiques et notamment la consistance d'une éponge ou d'une sphère souple. Il s'agit plus particulièrement d'un élastomère d'organopolysiloxane réticulé. L'élastomère est avantageusement un élastomère non émulsionnant c'est-à-dire un élastomère d'organopolysiloxane ne contenant pas de chaîne hydrophile, et en particulier ne contenant pas de motifs polyoxyalkylène (notamment polyoxyéthylène ou polyoxypropylène), ni de motif polyglycéryle.

En particulier, l'élastomère de silicone utilisé dans la présente invention peut être choisi parmi des Dimethicone Crosspolymer (Nom INCI), Vinyl Dimethicone Crosspolymer (Nom INCI), Dimethicone/Vinyl Dimethicone Crosspolymer (Nom INCI), Dimethicone Crosspolymer-3 (Nom INCI).

Les particules d'élastomères d'organopolysiloxane peuvent être véhiculées sous forme d'un gel constitué d'un organopolysiloxane élastomérique inclus dans au moins une huile hydrocarbonée et/ou une huile siliconée. Des élastomères non émulsionnants sont notamment décrits dans les brevets EP 242 219, EP 285 886, EP 765 656 et dans la demande JP-A-61-194009.

On peut citer notamment les composés de nom INCI suivants :
- Dimethicone/Vinyl Dimethicone Crosspolymer, tels que « USG-105 » et « USG-107A » de la société Shin Etsu; «DC9506» et « DC9701 » de la société Dow Corning ;
- Dimethicone/Vinyl Dimethicone Crosspolymer (and) Dimethicone, tels que « KSG-6 » et « KSG-16 » de la société Shin Etsu ;
- Dimethicone/Vinyl Dimethicone Crosspolymer (and) Cyclopentasiloxane, tel que « KSG-15 » ;
- Cyclopentasiloxane (and) Dimethicone Crosspolymer, tels que « DC9040 », « DC9045 » et « DC5930 » de la société Dow Corning ;
- Dimethicone (and) Dimethicone Crosspolymer, tel que « DC9041 » de la société Dow Corning ;
- Dimethicone (and) Dimethicone Crosspolymer, tel que «Dow Corning EL-9240® silicone elastomer blend » de la société Dow Corning (mélange de polydiméthylsiloxane réticulé avec hexadiène/polydiméthysiloxane (2 cSt)) ;
- C₄-₂₄ Alkyl Dimethicone/DivinylDimethicone Crosspolymer, tel que NuLastic Silk MA par la société Alzo ;
- Dimethicone/Vinyl Dimethicone Crosspolymer (and) Dimethicone, tels que « KSG-6 » et « KSG-16 » de la société Shin Etsu ;
- Dimethicone (and) Dimethicone Crosspolymer, tel que « DC9041 » de la société Dow Corning ;
- Dimethicone (and) Dimethicone Crosspolymer, tel que «Dow Corning EL-9240® silicone elastomer blend » de la société Dow Corning (mélange de polydiméthylsiloxane réticulé par Hexadiène/Polydiméthysiloxane (2 cSt)) ; et
- le mélange de Polydiméthylsiloxane réticulé par Hexadiène/Polydiméthylsiloxane (5cst) commercialisé sous la dénomination DC 9041 par la société Dow Corning.

Les particules d'élastomères d'organopolysiloxane peuvent également être utilisées sous forme de poudre, on peut notamment citer les poudres vendues sous les dénominations « Dow Corning 9505 Powder », « Dow Corning 9506 Powder » par la société Dow Corning, ces poudres ont pour nom INCI : dimethicone/vinyl dimethicone crosspolymer. La poudre d'organopolysiloxane peut également être enrobée de résine silsesquioxane, comme décrit par exemple dans le brevet US 5,538,793. De telles poudres d'élastomères sont vendues sous les dénominations « KSP-100 », « KSP-101 », « KSP-102 », « KSP-103 », « KSP-104 », «KSP-105» par la société Shin Etsu, et ont pour nom INCI : vinyl dimethicone/methicone silsesquioxane Crosspolymer.

d) Par « polymère semi-cristallin », on entend au sens de l'invention, des polymères comportant une partie cristallisable et une partie amorphe et présentant une température de changement de phase réversible du premier ordre, en particulier de fusion (transition solide-liquide). La partie cristallisable est soit une chaîne latérale (ou chaîne pendante), soit une séquence dans le squelette. De préférence, les polymères semi-cristallins de l'invention sont d'origine synthétique. Selon un mode de réalisation préféré, le polymère semi-cristallin est choisi parmi :
- les homopolymères et copolymères comportant des motifs résultant de la polymérisation de un ou plusieurs monomères porteurs de chaîne(s) latérale(s) hydrophobe(s) cristallisable(s),
- les polymères portant dans le squelette au moins une séquence cristallisable,
- les polycondensats de type polyester, aliphatique ou aromatique ou aliphatique/aromatique,
- les copolymères d'éthylène et de propylène préparés par catalyse métallocène, et
- les copolymères acrylates/silicone.

Les polymères semi-cristallins utilisables dans l'invention peuvent être choisis en particulier parmi :
- les copolymères séquencés de polyoléfines à cristallisation contrôlée, dont les monomères sont décrits dans EP 0 951 897,
- les polycondensats et notamment de type polyester, aliphatique ou aromatique ou aliphatique/aromatique,
- les copolymères d'éthylène et de propylène préparés par catalyse métallocène,
- les homo- ou co-polymères portant au moins une chaîne latérale cristallisable et les homo- ou co-polymères portant dans le squelette au moins une séquence cristallisable, comme ceux décrits dans le document US 5,156,911, tels que les (C₁₀-C₃₀)alkyle polyacrylates correspondant aux Intelimer® de la société Landec décrits dans la brochure « Intelimer® polymers », Landec IP22 (Rev. 4-97) et par exemple le produit Intelimer® IPA 13-1 de la société Landec, qui est un polyacrylate de stéaryle de poids moléculaire d'environ 145 000 et dont la température de fusion est égale à 49 °C,
- les homo- ou co-polymères portant au moins une chaîne latérale cristallisable en particulier à groupement(s) fluoré(s), tels que décrits dans le document WO 01/19333,
- les copolymères acrylates/silicone, tels que les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier les copolymères commercialisés par la société SHIN-ETSU sous les dénominations KP-561 (nom CTFA : acrylates/dimethicone), KP-541 (nom CTFA : acrylates/dimethicone and Isopropyl alcohol), KP-545 (nom CTFA : acrylates/dimethicone and Cyclopentasiloxane),
   et leurs mélanges.

Le gélifiant polymérique peut également être un ester de dextrine. Il s'agit de préférence d'au moins un ester de dextrine et d'acide gras, de préférence en C12 à C24, en particulier en C14 à C18, ou leurs mélanges. De préférence, l'ester de dextrine est choisi parmi le myristate de dextrine et/ou le palmitate de dextrine, et leurs mélanges. Selon un mode de réalisation préféré, celui-ci peut par exemple être choisi parmi ceux commercialisés sous les dénominations Rheopearl TL® ou Rheopearl KL® ou Rheopearl® KL2 par la société Chiba Flour Milling et Rheopearl MKL-2 par la société Chiba Flour Milling.

Selon un mode de réalisation, une composition selon l'invention comprend au moins un ingrédient additionnel étant au moins :
- un gélifiant hydrophile choisi parmi les silices pyrogénées, les gélifiants polymériques synthétiques, les gélifiants polymériques naturels ou d'origine naturelle comme par exemple les polysaccharides non amylacés, les amidons natifs, les amidons modifiés, les amidons greffés par un polymère acrylique, les amidons hydrolysés greffés par un polymère acrylique, les polymères à base d'amidon, de gomme et de dérivé cellulosique, les homo- ou copolymères d'acides acrylique ou méthacrylique, leurs sels ou leurs esters, les copolymères acide polyacryliques/acrylates d'alkyle, les copolymères AMPS/méthacrylates d'alkyle polyoxyéthylénés, réticulés ou non réticulés, les polymères de chitine ou de chitosane anioniques, cationiques, amphotères ou non-ioniques, les polymères de cellulose, distincts de l'alkylcellulose,choisis parmi l'hydroxyéthylcellulose, l'hydroxypropylcellulose, l'hydroxyméthylcellulose, l'éthylhydroxyéthylcellulose, la carboxyméthylcellulose, ainsi que les dérivés quaternisés de la cellulose, les polymères vinyliques et les polymères associatifs anioniques, cationiques, non-ioniques ou amphotères, les polymères d'origine naturelle modifiés ou non modifiés, les alginates et les carraghénanes, les glycoaminoglycanes, les acides hyaluroniques et leurs dérivés, l'acide désoxyribonucléique, les muccopolysaccharides, les chondroïtines sulfate, et leurs mélanges ; et/ou
- un gélifiant lipophile choisi parmi les gélifiants particulaires, les élastomères d'organopolysiloxane, les polymères semi-cristallins, les copolymères séquencés hydrocarbonés, les esters de dextrine, les polymères à liaison hydrogène et leurs mélanges.

Une composition selon l'invention peut avantageusement comprendre de 0,01 % à 8 % en poids de gélifiant(s), de préférence de 0,1 % à 3 % en poids, par rapport au poids total de la composition.

### Polymères filmogènes hydrophobes

Une composition selon l'invention peut en outre comprendre à titre d'ingrédient additionnel au moins un polymère filmogène hydrophobe.

Au sens de la présente invention, on entend désigner par « *polymère filmogène hydrophobe* », un polymère filmogène dénué d'une affinité pour l'eau et à ce titre ne se prêtant pas une formulation à l'état de soluté dans un milieu aqueux. En particulier, par polymère hydrophobe, on entend un polymère ayant une solubilité dans l'eau à 25 °C inférieure à 1 % en poids.

Par polymère «*filmogène*», on entend un polymère apte à former à lui seul ou en présence d'un agent auxiliaire de filmification, un dépôt macroscopiquement continu sur un support, notamment sur la peau et/ou les ongles, et de préférence un dépôt cohésif, et mieux encore un dépôt dont la cohésion et les propriétés mécaniques sont telles que ledit dépôt peut être isolable et manipulable isolément, par exemple lorsque ledit dépôt est réalisé par coulage sur une surface antiadhérente comme une surface téflonnée ou siliconée.

En particulier, le polymère filmogène hydrophobe est un polymère choisi parmi le groupe comprenant :
- les polymères filmogènes solubles dans un milieu solvant organique, en particulier les polymères liposolubles ; cela signifie que le polymère est soluble ou miscible dans le milieu organique et va former une seule phase homogène lorsqu'il sera incorporé dans le milieu ; et
- les polymères filmogènes dispersibles dans un milieu solvant organique, cela signifie que le polymère forme une phase insoluble dans le milieu organique, le polymère restant stable et/ou compatible une fois incorporé dans ce milieu. En particulier, de tels polymères peuvent se présenter sous la forme de dispersions non aqueuses de particules de polymères, de préférence des dispersions dans les huiles siliconées ou hydrocarbonées ; dans un mode de réalisation, les dispersions non aqueuse de polymère comprennent des particules polymères stabilisées sur leur surface par au moins un agent stabilisant ; ces dispersions non aqueuses sont souvent appelées « NAD (non-aqueous dispersions) ».

Comme polymère filmogène hydrophobe, on peut citer notamment les homopolymères et les copolymères de composé à motif éthylénique, les polymères et copolymères acryliques, les polyuréthanes, les polyesters, les polymères siliconés tels que les polymères à squelette organique non-siliconé greffé par des monomères contenant un polysiloxane, les polyisoprènes.

Une composition selon l'invention peut comprendre de 1 % à 30 % en poids, de préférence de 2 % à 25 % en poids et encore plus préférentiellement de 5 % à 20 % en poids de polymère(s) filmogène(s) hydrophobe(s), par rapport au poids total de la composition.

Comme polymères filmogènes hydrophobes convenant tout particulièrement à l'invention peuvent notamment être cités les polymères filmogènes lipodispersibles sous forme de dispersions non aqueuses de particules de polymères (NAD), les copolymères éthyléniques séquencés, les polymères vinyliques comprenant au moins un motif dérivé de dendrimère carbosiloxane, les copolymères silicone acrylate et leurs mélanges, de préférence, les polymères filmogènes lipodispersibles sous forme de dispersions non aqueuses de particules de polymères (NAD).

### 1. Polymères filmogènes lipodispersibles sous forme de dispersions non aqueuses de particules de polymère, encore appelées «NAD»

Selon une autre variante de réalisation, une composition selon l'invention peut comprendre, à titre de polymère filmogène hydrophobe, au moins un polymère choisi parmi les polymères filmogènes lipodispersibles sous forme de dispersions non aqueuses de particules de polymère, encore appelées « NAD ».

Comme dispersion non aqueuse de polymère filmogène hydrophobe, on peut utiliser les dispersions de particules d'un polymère éthylénique greffé, de préférence acrylique, dans une phase huileuse liquide par exemple, sous forme de particules, stabilisées en surface, dispersées dans la phase grasse liquide.

La dispersion de particules de polymères stabilisées en surface peut être fabriquée comme décrite dans le document WO 04/055081.

### 2. Copolymère éthylénique séquencé

Selon un premier mode de réalisation de l'invention, le polymère filmogène hydrophobe est un copolymère éthylénique séquencé, contenant au moins une première séquence ayant une température de transition vitreuse (T_{g}) supérieure ou égale à 40 °C et étant issue en totalité ou en partie de un ou plusieurs premiers monomères, qui sont tels que l'homopolymère préparé à partir de ces monomères a une température de transition vitreuse supérieure ou égale à 40 °C, et au moins une deuxième séquence ayant une température de transition vitreuse inférieure ou égale à 20 °C et étant issue en totalité ou en partie de un ou plusieurs deuxièmes monomères, qui sont tels que l'homopolymère préparé à partir de ces monomères a une température de transition vitreuse inférieure ou égale à 20 °C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2.

Des polymères de ce type convenant à l'invention sont décrits dans le document EP 1 411 069.

A titre d'exemple de tels polymères, on peut plus particulièrement citer le MEXOMERE PAS® (Copolymère Acide acrylique/Acrylate d'isobutyle/Acrylate d'isobornyle dilué à 50 % dans l'isododécane) commercialisé par la société CHIMEX.

### 3. Polymère vinylique comprenant au moins un motif dérivé de dendrimère carbosiloxane

Selon un mode de réalisation particulier, une composition utilisée selon l'invention peut comprendre, à titre de polymère filmogène hydrophobe, au moins un polymère vinylique comprenant au moins un motif dérivé de dendrimère carbosiloxane.

Le polymère vinylique utilisé selon l'invention possède notamment un squelette et au moins une chaîne latérale, laquelle comprend un motif dérivé de dendrimère carbosiloxane présentant une structure de dendrimère carbosiloxane.

En particulier, on pourra utiliser les polymères vinyliques comprenant au moins un motif dendrimère carbosiloxane tels que décrits dans les demandes WO03/045337 et EP 963 751 de la société Dow Corning.

Le terme « structure de dendrimère carbosiloxane » dans le contexte de la présente invention représente une structure moléculaire possédant des groupes ramifiés ayant des masses moléculaires élevées, ladite structure ayant une régularité élevée dans la direction radiale en partant de la liaison au squelette. De telles structures de dendrimère carbosiloxane sont décrites sous la forme d'un copolymère siloxane-silylalkylène fortement ramifié dans la demande de brevet japonais mise à l'inspection publique Kokai 9-171 154.

Un polymère vinylique ayant au moins un motif dérivé de dendrimère carbosiloxane possède une chaîne moléculaire latérale contenant une structure de dendrimère carbosiloxane, et peut être issu de la polymérisation :
(A) de 0 à 99,9 parties en poids d'un monomère vinylique ; et
(B) de 100 à 0,1 parties en poids d'un dendrimère carbosiloxane contenant un groupe organique polymérisable à l'aide de radicaux, représenté par la formule générale : 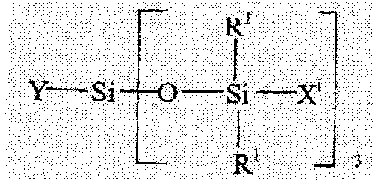 dans laquelle Y représente un groupe organique polymérisable à l'aide de radicaux, R¹ représente un groupe aryle ou un groupe alkyle possédant de 1 à 10 atomes de carbone, et Xⁱ représente un groupe silylalkyle qui, lorsque i = 1, est représenté par la formule : 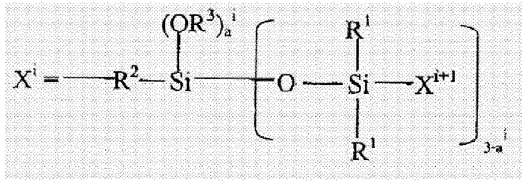 dans laquelle R¹ est tel que défini ci-dessus, R² représente un groupe alkylène possédant de 2 à 10 atomes de carbone, R³ représente un groupe alkyle possédant de 1 à 10 atomes de carbone, Xⁱ⁺¹ représente un atome d'hydrogène, un groupe alkyle possédant de 1 à 10 atomes de carbone, un groupe aryle, ou le groupe silylalkyle défini ci-dessus avec i = i + 1 ; i est un nombre entier de 1 à 10 qui représente la génération dudit groupe silylalkyle, et aⁱ est un nombre entier de 0 à 3 ;
   où ledit groupe organique polymérisable à l'aide de radicaux contenu dans le composant (A) est choisi parmi :
   - des groupes organiques contenant un groupe méthacrylique ou un groupe acrylique et qui sont représentés par les formules : 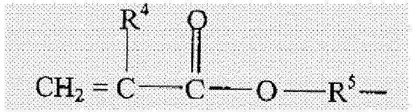 et 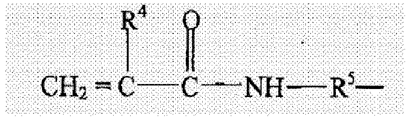 dans lesquelles R⁴ représente un atome d'hydrogène ou un groupe alkyle, R⁵ représente un groupe alkylène possédant de 1 à 10 atomes de carbone ; et
   - des groupes organiques contenant un groupe styryle et qui sont représentés par la formule : 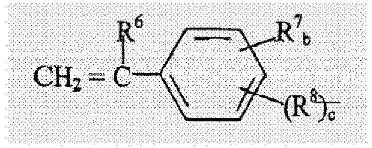 dans laquelle R⁶ représente un atome d'hydrogène ou un groupe alkyle, R⁷ représente un groupe alkyle possédant de 1 à 10 atomes de carbone, R⁸ représente un groupe alkylène possédant de 1 à 10 atomes de carbone, b est un nombre entier de 0 à 4, et c vaut 0 ou 1, de sorte que si c vaut 0, -(R⁸)_{c}- représente une liaison.

Le monomère de type vinyle qui est le composant (A) dans le polymère vinylique est un monomère de type vinyle qui contient un groupe vinyle polymérisable à l'aide de radicaux.

Il n'y a aucune limitation particulière en ce qui concerne un tel monomère.

Ce qui suit sont des exemples de ce monomère de type vinyle : le méthacrylate de méthyle, le méthacrylate d'éthyle, le méthacrylate de n-propyle, le méthacrylate d'isopropyle, ou un méthacrylate d'alkyle analogue inférieur ; le méthacrylate de glycidyle ; le méthacrylate de butyle, l'acrylate de butyle, le méthacrylate de n-butyle, le méthacrylate d'isobutyle, l'acrylate de tert-butyle, le méthacrylate de tert-butyle, le méthacrylate de n-hexyle, le méthacrylate de cyclohexyle, l'acrylate de 2-éthylhexyle, le méthacrylate de 2-éthylhexyle, le méthacrylate d'octyle, le méthacrylate de lauryle, l'acrylate de stéaryle, le méthacrylate de stéaryle, ou un méthacrylate analogue supérieur ; l'acétate de vinyle, le propionate de vinyle, ou un ester de vinyle d'acide gras analogue inférieur ; le caproate de vinyle, le 2-éthylhexoate de vinyle, le laurate de vinyle, le stéarate de vinyle, ou un ester d'acide gras analogue supérieur ; le styrène, le vinyltoluène, le méthacrylate de benzyle, le méthacrylate de phénoxyéthyle, la vinylpyrrolidone, ou des monomères vinyliques aromatiques analogues ; le méthacrylamide, le N-méthylolméthacrylamide, le N-méthoxyméthylméthacrylamide, l'isobutoxyméthoxyméthacrylamide, le N,Ndiméthylméthacrylamide, ou des monomères analogues de type vinyle qui contiennent des groupes amide ; le méthacrylate d'hydroxyéthyle, le méthacrylate de l'alcool hydroxypropylique, ou des monomères analogues de type vinyle qui contiennent des groupes hydroxyle ; l'acide acrylique, l'acide méthacrylique, l'acide itaconique, l'acide crotonique, l'acide fumarique, l'acide maléique, ou des monomères analogues de type vinyle qui contiennent un groupe acide carboxylique ; le méthacrylate de tétrahydrofurfuryle, le méthacrylate de butoxyéthyle, le méthacrylate de l'éthoxydiéthylèneglycol, le polyéthylèneglycolméthacrylate, le polypropylèneglycolmonométhacrylate, l'éther d'hydroxybutyle et de vinyle, l'éther de cétyle et de vinyle, l'éther de 2-éthylhexyle et de vinyle, ou un monomère analogue de type vinyle avec des liaisons éther ; le méthacryloxypropyltriméthoxysilane, le polydiméthylsiloxane ayant un groupe méthacrylique sur l'une de ses extrémités moléculaires, le polydiméthylsiloxane ayant un groupe styryle sur une de ses extrémités moléculaires, ou un composé analogue de silicone possédant des groupes insaturés ; le butadiène ; le chlorure de vinyle ; le chlorure de vinylidène ; le méthacrylonitrile ; le dibutylfumarate ; l'acide maléique anhydre ; l'acide succinique anhydre ; l'éther de méthacryle et de glycidyle ; un sel organique d'une amine, un sel d'ammonium, et un sel de métal alcalin de l'acide méthacrylique, de l'acide itaconique, de l'acide crotonique, de l'acide maléique, ou de l'acide fumarique ; un monomère insaturé polymérisable à l'aide de radicaux possédant un groupe acide sulfonique tel qu'un groupe styrène acide sulfonique ; un sel d'ammonium quaternaire dérivé de l'acide méthacrylique tel que le chlorure de 2-hydroxy-3-méthacryloxypropyltriméthylammonium ; et un ester de l'acide méthacrylique d'un alcool possédant un groupe amine tertiaire tel qu'un ester de l'acide méthacrylique et de la diéthylamine.

Les monomères de type vinyle multifonctionnels peuvent également être utilisés.

Ce qui suit représente des exemples de tels composés : le triméthacrylate de triméthylolpropane, le triméthacrylate de pentaérythritol, le diméthacrylate d'éthylèneglycol, le diméthacrylate de tétraéthylèneglycol, le polyéthylèneglycoldiméthacrylate, le diméthacrylate de 1,4-butanediol, le diméthacrylate de 1,6-hexanediol, le diméthacrylate de néopentylglycol, le triméthylolpropanetrioxyéthylméthacrylate, le diméthacrylate de tris-(2-hydroxyéthyl)isocyanurate, le triméthacrylate de tris-(2-hydroxyéthyl)isocyanurate, le polydiméthylsiloxane coiffé de groupes styryle possédant des groupes divinylbenzène sur les deux extrémités, ou des composés analogues de silicone possédant des groupes insaturés.

Pour faciliter la préparation de mélange de la matière première de produits cosmétiques, la masse moléculaire moyenne en nombre du polymère vinylique qui contient un dendrimère carbosiloxane, peut être choisie dans la plage entre 3 000 g/mol et 2 000 000 g/mol, de préférence entre 5 000 g/mol et 800 000 g/mol. Il peut être un liquide, une gomme, une pâte, un solide, une poudre, ou toute autre forme. Les formes préférées sont les solutions constituées par la dilution dans des solvants tels qu'une huile de silicone ou une huile organique, d'une dispersion, ou d'une poudre.

Un polymère vinylique contenu dans la dispersion ou la solution peut avoir une concentration dans une plage comprise entre 0,1 et 95 % en poids, de préférence entre 5 et 70 % en poids. Cependant, pour faciliter la manipulation et la préparation de mélange, la plage devrait être de préférence entre 10 et 60 % en poids.

Selon un mode préféré, un polymère vinylique convenant à l'invention peut être un des polymères décrits dans les exemples de la demande EP 0 963 751.

Selon un mode préféré de réalisation, un polymère vinylique greffé avec un dendrimère carbosiloxane peut être issu de la polymérisation :
(A) de 0,1 à 99 partie en poids d'un ou plusieurs monomère(s) acrylate ou méthacrylate ; et
(B) de 100 à 0,1 parties en poids d'un monomère acrylate ou méthacrylate d'un dendrimère carbosiloxane tri[tri(triméthylsiloxy)silyléthyl diméthylsiloxy]silylpropyle.

Selon un mode de réalisation, un polymère vinylique ayant au moins un motif dérivé de dendrimère carbosiloxane peut comprendre un motif dérivé de dendrimère carbosiloxane tri[tri(triméthylsiloxy)silyléthyl diméthylsiloxy]silylpropyle répondant à l'une des formules : 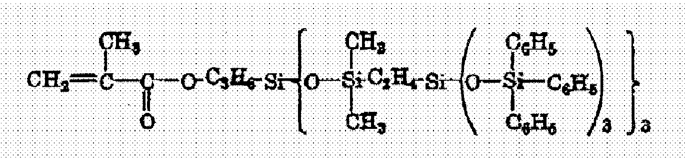 ou

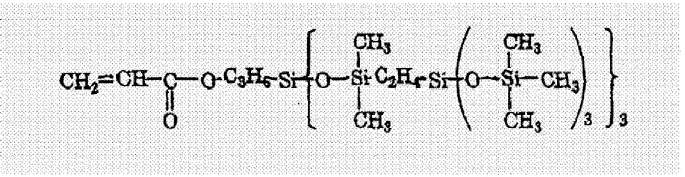

Selon un mode préféré, un polymère vinylique ayant au moins un motif dérivé de dendrimère carbosiloxane utilisé dans l'invention comprend au moins un monomère acrylate de butyle.

Selon un mode de réalisation, un polymère vinylique peut comprendre en outre au moins un groupement organique fluoré. Un polymère vinylique fluoré peut être un des polymères décrits dans les exemples de la demande WO 03/045337.

Selon un mode préféré de réalisation, un polymère vinylique greffé au sens de la présente invention peut être véhiculé dans une huile ou un mélange d'huile(s), de préférence volatile(s) en particulier, choisi(s) parmi les huiles de silicones et les huiles hydrocarbonées et leurs mélanges.

Selon un mode particulier de réalisation, une huile de silicone convenant à l'invention peut être la cyclopentasiloxane ou la cyclohexasiloxane.

Selon un autre mode particulier de réalisation, une huile hydrocarbonée convenant à l'invention peut être l'isododécane.

Les polymères vinyliques greffés avec au moins un motif dérivé de dendrimère carbosiloxane pouvant convenir particulièrement à la présente invention sont les polymères vendus sous les dénominations TIB 4-100, TIB 4-101, TIB 4-120, TIB 4-130, TIB 4-200, FA 4002 ID (TIB 4-202), TIB 4-220, FA 4001 CM (TIB 4-230) par la société Dow Corning. De préférence, on utilisera les polymères vendus sous les dénominations FA 4002 ID (TIB 4-202), et FA 4001 CM (TIB 4-230) par la société Dow Corning

De préférence, le polymère vinylique greffé avec au moins un motif dérivé de dendrimère carbosiloxane utilisable dans une composition de l'invention est un copolymère acrylate/polytriméthylsiloxyméthacrylate, notamment celui commercialisé dans l'isododécane sous la dénomination Dow Corning FA 4002 ID silicone acrylate par la société Dow Corning.

### 4. Copolymères silicone acrylate

Selon un mode de réalisation particulier, une composition utilisée selon l'invention peut comprendre, à titre de polymère filmogène hydrophobe, au moins un copolymère comportant des groupements carboxylates et des groupements polydiméthylsiloxanes.

Par « copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Selon un mode préféré de réalisation de l'invention, les monomères sous forme d'esters sont plus particulièrement choisis parmi les acrylates et méthacrylates d'alkyle linéaire ou ramifié de préférence en C₁-C₂₄ et mieux en C₁-C₂₂, le radical alkyle étant préférentiellement choisi parmi les radicaux méthyle, éthyle, stéaryle, butyle, éthyl-2-hexyle, et leurs mélanges.

Ainsi, selon un mode particulier de réalisation de l'invention, le copolymère comprend comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

Dans la présente demande, on entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées pour obtenir le copolymère utilisé selon l'invention comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe CH₂ = CR₁ - CO - O - R₂, où Ri représente un hydrogène ou un groupe méthyle, et R₂ représente -CH₂-, -(CH₂)ₙ- avec n = 3, 5, 8 ou 10, -CH₂-CH(CH₃)-CH₂-, -CH₂-CH₂-O-CH₂-CH₂-, -CH₂-CH₂-O-CH₂-CH₂-CH(CH₃)-CH₂-, -CH₂-CH₂-O-CH₂ CH₂-O-CH₂-CH₂-CH₂-.

Les copolymères utilisés dans la composition de l'invention sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids moléculaire allant d'environ 3 000 g/mol à 200 000 g/mol et de préférence d'environ 5 000 g/mol à 100 000 g/mol.

Le copolymère utilisé dans la composition de l'invention peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables dans la composition de l'invention, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier comme copolymère utilisable dans la composition de l'invention, les copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA : acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30°C.

On peut également citer le copolymère greffé de polyacrylique et diméthylpolysiloxane dissous dans l'isododécane commercialisé par la société Shin Etsu sous la dénomination KP-550.

### Ingrédients choisis parmi les actifs cosmétiques, parfums et les matières colorantes

Selon un mode de réalisation, l'ingrédient additionnel compris dans une composition selon l'invention est au moins un composé choisi parmi les actifs cosmétiques ; les vitamines ; les filtres anti-UV ; les matières colorantes, de préférence choisies parmi les pigments et les nacres ; les parfums ; et leurs mélanges.

### Actifs cosmétiques

Un actif cosmétique selon la présente invention peut être choisi notamment parmi les agents hydratants (également appelé agent humectant), les filtres UV, les agents cicatrisants, les agents blanchissants ou dépigmentants, les agents anti-transpirants et/ou les agents anti-âge. On peut également considérer à titre d'actifs cosmétiques certains tensioactifs anioniques utilisés dans des produits de nettoyage de la peau et/ou des ongles, et notamment de la peau du visage et/ou du corps.

Comme agents hydratants, on peut citer notamment les polyols ; les céramides ; la DHEA et ses dérivés ; le coenzyme Q10 ; et l'acide hyaluronique et ses dérivés.

Comme exemples d'agents cicatrisants, on peut citer notamment l'allantoïne, l'urée, certains acides aminés comme l'hydroxyproline, l'arginine et la sérine.

Comme exemples d'agents anti-transpirants, on peut citer les sels astringents comme les sels d'aluminium et/ou les sels de zirconium.

Comme exemples d'agents anti-âge, on peut citer notamment les agents kératolytiques ou prodesquamants, par exemple les α-hydroxy-acides, les β-hydroxy-acides, les α-céto-acides, les β-ceto-acides, les rétinoïdes et leurs esters, le rétinal, l'acide rétinoïque et ses dérivés.

Comme tensioactifs ioniques convenant à titre d'actifs cosmétiques on peut notamment citer tensioactifs anioniques choisis parmi les alkylsulfates ; les alkyléthersulfates ; les acylglutamates ; les acylsarcosinates ; les acyllactylates ; les alkyléthercarboxylates ; les acyliséthionates tels que les cocoyl-iséthionates et/ou les lauroyl-iséthionates.

Selon une variante particulière de réalisation, une composition selon l'invention contient au moins à titre d'ingrédient distinct du phyllosilicate synthétique, de l'acide hyaluronique, réticulé et/ou non réticulé, un de ses dérivés. Par dérivé de l'acide hyaluronique, on entend préférentiellement l'un de ses sels, en particulier son sel de sodium. De préférence, il s'agit du hyaluronate de sodium (NaHA).

Une composition selon l'invention peut ainsi avantageusement comprendre de 0,1% à 10% en poids d'acide hyaluronique ou de l'un de ses dérivés, de préférence de 0,5% à 5% en poids, par rapport au poids total de la composition.

De façon préférée, une composition selon l'invention peut comprendre de 0,001 % à 30 % en poids, de préférence de 0,01 à 20 % en poids, ou encore mieux de 0,01 % à 10 % en poids, de préférence allant de 0,01 % à 5 % en poids, en poids d'actif(s) cosmétique(s), par rapport au poids total de la composition.

### Parfums

Une composition selon l'invention peut également comprendre un parfum notamment sous la forme d'une huile essentielle ou d'un mélange d'huiles essentielles.

### Matières colorantes

Les compositions conformes à l'invention comprennent avantageusement au moins une matière colorante.

Cette (ou ces) matière(s) colorante(s) est (ou sont) de préférence choisie parmi les matières colorantes pulvérulentes, les colorants liposolubles, les colorants hydrosolubles, et leurs mélanges.

De préférence, les compositions selon l'invention comportent au moins une matière colorante pulvérulente. Les matières colorantes pulvérulentes peuvent être choisies parmi les pigments et les nacres, de préférence parmi les pigments.

Les pigments peuvent être blancs ou colorés, minéraux et/ou organiques, enrobés ou non. On peut citer, parmi les pigments minéraux, des oxydes métalliques, en particulier le dioxyde de titane, éventuellement traité en surface, les oxydes de zirconium, de zinc ou de cérium, ainsi que les oxydes de fer, de titane, ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

De préférence, les pigments contenus dans les compositions selon l'invention sont choisis parmi des oxydes métalliques.

Ces matières colorantes peuvent être présentes en une teneur allant de 0,01 à 30 % en poids par rapport au poids total de la composition, en particulier de 3 à 22% en poids par rapport au poids total de la composition.

De préférence, la (les) matières colorante(s) est (sont) choisi(s) parmi un ou plusieurs oxyde(s) métallique(s) présent(s) à une teneur supérieure ou égale à 2 % en poids par rapport au poids total de la composition, avantageusement comprise inclusivement entre 3 et 25 % en poids par rapport au poids total de la composition.

### Vitamines

Une composition selon l'invention peut également comprendre au moins une vitamine choisie par exemple parmi les vitamines A, B3, PP, B5, E, K1 et/ou C et les dérivés de ces vitamines et notamment leurs esters et leurs mélanges.

### Conservateurs

Par « conservateur », on entend désigner une substance d'origine naturelle ou synthétique incorporée à une composition, notamment cosmétique, dans le but d'éviter les altérations d'ordre chimique (oxydation) ou microbiologique.

Les conservateurs selon l'invention peuvent être choisis parmi les conservateurs antimicrobiens, les conservateurs antioxydants, et leurs mélanges. Le choix de ces composés relève clairement des compétences de l'homme de l'art.

Bien entendu, tous les agents ou composés additionnels susmentionnés sont différents des phyllosilicates synthétiques selon l'invention.

### MILIEU PHYSIOLOGIQUEMENT ACCEPTABLE

Comme exposé ci-avant, une composition selon l'invention peut être avantageusement cosmétique ou dermatologique.

Dans ce mode de réalisation particulier, une composition selon l'invention étant destinée à une application topique sur la peau et/ou les ongles, elle contient un milieu physiologiquement acceptable.

Au sens de la présente invention, on entend par "milieu physiologiquement acceptable", un milieu compatible avec la peau et/ou les ongles.

Ainsi, le milieu physiologiquement acceptable est notamment un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Préférentiellement, une composition de l'invention est une composition cosmétique.

Au vu de ce qui précède, une composition selon l'invention est destinée à une administration par voie topique, c'est-à-dire par application en surface de la peau et/ou des ongles.

Ainsi, une composition selon l'invention peut être sous la forme de produits de soin de la peau ou des semi-muqueuses tels qu'une composition de protection, de traitement ou de soin pour le visage, pour les lèvres, pour les mains, pour les ongles voire les faux-ongles, pour les pieds, pour les plis anatomiques ou pour le corps (par exemple crèmes de jour, crème de nuit, crème démaquillante, base de maquillage, composition anti-solaire, lait corporel de protection ou de soin, lait après-solaire, lotion, gel ou mousse pour le soin de la peau, sérum, masque, composition de bronzage artificiel, composition après-rasage, produit pour l'hygiène et le nettoyage de la peau et/ou des ongles.

La composition selon l'invention peut se présenter sous toutes les formes galéniques normalement utilisées dans les domaines cosmétique et dermatologique.

Elle peut être notamment sous forme d'une solution aqueuse, hydroalcoolique, éventuellement gélifiée, d'une dispersion du type lotion éventuellement bi ou triphasée, d'une émulsion huile-dans-eau ou eau-dans-huile ou multiple, d'un gel aqueux, d'une dispersion d'huiles dans une phase aqueuse. Elle peut être également de consistance solide ou pâteuse.

Selon un mode de réalisation particulier, une composition selon l'invention peut encore avantageusement se présenter sous une formulation dite de type gel-gel, c'est-à-dire figurée par une composition comprenant :
- au moins une phase aqueuse gélifiée par au moins un gélifiant hydrophile; et
- au moins une phase huileuse gélifiée par au moins un agent gélifiant lipophile, les deux phases y formant un mélange macroscopiquement homogène.

Un tel mode de réalisation est notamment illustré en exemple 4 ci-après.

Selon ce mode de réalisation particulier, le gel aqueux ou hydroalcoolique de phyllosilicate synthétique peut être utilisé à titre de gélifiant hydrophile. En effet, de par ses propriétés spécifiques, un gel aqueux ou hydroalcoolique de phyllosilicate synthétique conforme à l'invention peut jouer le rôle de charge et/ou de gélifiant hydrophile au sein d'une composition selon l'invention.

Selon un mode de réalisation, une composition de l'invention se présente sous la forme d'une composition de nettoyage de la peau et/ou des ongles, notamment la peau du corps ou du visage, en particulier du visage.

Selon un mode de réalisation, une composition de l'invention peut avantageusement se présenter sous la forme d'une composition de soin de la peau et/ou des ongles, notamment la peau du corps ou du visage, en particulier du visage.

Selon un autre mode de réalisation, une composition de l'invention peut avantageusement se présenter sous la forme d'une composition de maquillage de la peau et/ou des ongles, notamment la peau du corps ou du visage, en particulier du visage.

Ainsi, selon un sous mode de ce mode de réalisation, une composition de l'invention peut avantageusement se présenter sous la forme d'une composition de base de maquillage pour le maquillage.

Une composition de l'invention peut avantageusement se présenter sous la forme d'un fond de teint.

Selon un autre sous mode de ce mode de réalisation, une composition de l'invention peut avantageusement se présenter sous la forme d'une composition de maquillage de la peau, du corps ou du visage, et notamment du visage. Il peut ainsi s'agir d'un fard à paupières ou d'un fard à joues.

Selon un mode de réalisation, une composition selon l'invention peut se présenter sous la forme d'une composition de soin et/ou de maquillage de la peau et/ou des ongles, du corps ou du visage, en particulier du visage, et est de préférence un fond de teint, un fard à joues ou à paupières.

Selon encore un autre sous mode de ce mode de réalisation, une composition de l'invention peut avantageusement se présenter sous la forme d'un produit dédiée au soin et/ou maquillage des lèvres et est de préférence un stick, tel qu'un rouge à lèvres, un vernis ou un gloss. Le phyllosilicate synthétique sous forme de gel aqueux selon l'invention présente l'avantage de conduire à la formulation de sticks émulsions utilisables dans le domaine du soin et du maquillage.

Selon encore un autre sous mode de ce mode de réalisation, une composition de l'invention peut avantageusement se présenter sous la forme d'un produit dédiée au soin et/ou maquillage des ongles, ou des faux-ongles, et est de préférence un vernis.

De telles compositions sont notamment préparées selon les connaissances générales de l'homme de l'art.

### PROCEDES ET UTILISATIONS

Dans tous les cas, une composition selon l'invention peut être préparée selon des méthodes connues de l'homme du métier. Bien entendu, le procédé de préparation des compositions selon l'invention dépend du type de formulation souhaitée.

Les phyllosilicates synthétiques selon l'invention étant mis en œuvre sous forme d'un gel aqueux ou hydroalcoolique, leur incorporation est donc privilégiée dans la phase aqueuse.

Comme indiqué précédemment, le présent texte décrit un procédé de maquillage et/ou de soin de la peau et/ou des ongles comprenant une étape d'application sur ladite peau et/ou lesdits ongles d'une composition comprenant, dans un milieu physiologiquement acceptable, au moins un gel aqueux ou hydroalcoolique de phyllosilicate synthétique de formule Mg₃Si₄O₁₀(OH)₂ conforme à l'invention.

Selon un mode de réalisation, la composition mise en œuvre est telle que décrite précédemment.

La présente invention concerne ainsi un procédé de maquillage et/ou de soin de la peau et/ou des ongles comprenant au moins une étape d'application sur ladite peau et/ou lesdits ongles d'une composition comprenant, dans un milieu physiologiquement acceptable, au moins un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, ladite composition comprenant une phase aqueuse formée au moins en partie par ledit gel aqueux ou hydroalcoolique de phyllosilicate synthétique.

Avantageusement, ce procédé peut être dédié à procurer un résultat maquillage matifiant et/ou homogène du teint de la peau après application.

Ce procédé peut également être dédié à faciliter l'étalement sur la peau de la composition et à conduire à un dépôt homogène d'actifs sur la peau après application. Ces actifs peuvent notamment être choisis parmi les actifs cosmétiques ; les vitamines ; les filtres anti-UV ; les matières colorantes, de préférence choisies parmi les pigments et les nacres ; les parfums ; et leurs mélanges.

De préférence, un procédé ou une utilisation tel que décrit ci-dessus peut mettre en œuvre une composition selon l'invention telle que décrite précédemment.

Dans toute la description, y compris les revendications, l'expression « comportant un » doit être comprise comme étant synonyme de « comportant au moins un », sauf si le contraire est spécifié.

Les expressions « compris entre... et ... » et « allant de ... à ... » doivent se comprendre bornes incluses, sauf si le contraire est spécifié.

Les exemples et figures qui suivent sont présentés à titre illustratif et non limitatif de l'invention.

### EXEMPLES

### EXEMPLE 1 : Préparation d'un phyllosilicate synthétique sous forme de gel aqueux convenant à l'invention

Un gel aqueux de phyllosilicate synthétique selon l'invention est préparé selon la technologie décrite dans l'exemple 1 de la demande FR 2 977 580 de la page 21, ligne 26 à la page 22, ligne 29. Il a ainsi été procédé jusqu'à la formation de l'hydrogel sans étape de séchage et de lyophilisation.

C'est ce gel aqueux qui est utilisé dans tous les exemples ci-après.

L'analyse du diffractogramme des rayons X a été effectuée à l'aide des matériel et méthode utilisés pour les analyses en diffraction des rayons X qui sont détaillés dans la demande FR 2 977 580.

Une raie de diffraction caractéristique à 9,77 Å est observée.

### EXEMPLE 2 : Influence de la nature de charge sur l'effet matifiant et homogénéisant après application d'une composition cosmétique de type émulsion directe (i.e. huile-dans-eau)

### 2.1. Compositions testées

Le phyllosilicate sous forme de gel aqueux a été introduit dans la phase aqueuse d'une émulsion huile-dans-eau telle que définie ci-après.

Les matières de la phase A1 sont tout d'abord pesées puis mises sous agitation Moritz sans chauffer. La phase A2 est ensuite ajoutée, puis le mélange est laissé sous agitation jusqu'à obtenir un mélange homogène.

On ajoute alors les phases A3, A4 et A5.

La phase B est mise à fondre à 68 °C puis est ajoutée dans le mélange A.

Le mélange ainsi obtenu est émulsionné sous vive agitation pendant 10 minutes.

On laisse enfin refroidir à 30 °C puis les phases C et D sont ajoutées.

La tare est enfin faite à l'eau.

Les compositions suivantes ont ainsi été préparées.

Les pourcentages indiqués dans ce tableau sont des pourcentages en poids du poids total de la composition correspondante.

| | **Matières premières** | **Références commerciales** | **Composition 1 (Témoin)** | **Composition 2 (Selon l'invention)** | **Composition 3 (comparatif)** |
|---|---|---|---|---|---|
| **A1** | Eau désionisée micro biologiquement propre | | Qsp 100 | Qsp 100 | Qsp 100 |
| | 2 -Phénoxyéthanol | | 0,40 % | 0,40 % | 0,40 % |
| | Acide éthylène diamine tétracétique, sel disodique, 2 H2O | Dissolvine NA2-S® (Akzo Nobel) | 0,05 % | 0,05 % | 0,05 % |
| **A2** | 1,3-butylène glycol | | 5,00 % | 5,00 % | 5,00 % |
| | Gomme de Xanthane | Rhodicare XC® (Rhodia) | 0,20 % | 0,00 % | 0,00 % |
| **A3** | **Gel de phyllosilicate synthétique selon l'invention (11% MA) dans l'eau** | | **0,00 %** | **1,80 % (soit 0,20% MA*)** | **0,00 %** |
| **A4** | Pigments | Base pigmentaire de NaI (Sunpuro) | 17,06 % | 17,06 % | 17,06 % |
| **A5** | Chlorhexidine digluconate en solution aqueuse | | 0,25 % | 0,25 % | 0,25 % |
| **B** | Mélange mono/distearate de glycéryle / stéarate de polyéthylène glycol (100 OE) | TEGO CARE 180® (Evonik Goldschmidt) | 3,00 % | 3,00 % | 3,00 % |
| | Alcool cétylstéarylique (C16/C18 50/50) | | 1,00 % | 1,00 % | 1,00 % |
| | Isoparaffine (6-8 Moles d'isobutylène) hydrogénée | Parléam ® (Nof Corporation) | 10,0 % | 10,0 % | 10,0 % |
| | Polydiméthylsiloxane (Viscosité: 10 CST) | Element 14 PDMS 10-A® (Momentive Performance Materials) | 5,00% | 5,00 % | 5,00 % |
| | Mélange de polydiméthylsiloxane réticulé et de polydiméthylsiloxane (6 CST) (24/76) | KSG 16® (Shin Etsu) | 7,00 % | 7,00 % | 7,00 % |
| **C** | **Talc naturel** | Luzenac ® Pharma M | **0,00 %** | **0,00 %** | **0,20 %** |
| **D** | Alcool éthylique absolu dénaturé | | 5,00 % | 5,00 % | 5,00 % |
| **TOTAL** | | | 100 | 100 | 100 |

| | | | | | |
|---|---|---|---|---|---|
| *MA : Matière Active | | | | | |

### 2.2. Evaluation de la viscosité des compositions 1 à 3

### Protocole de mesure de la viscosité :

### Mode opératoire

### 1. Thermostatisation

Une viscosité n'est significative qu'à une température donnée.

La mesure est effectuée avec un ensemble : produit à analyser - godet - corps de mesure. C'est cet ensemble qui doit être à la température de consigne T °C +/- 0.5 °C (en général 25 °C).

### 2. Réglage du zéro

Cette opération sera effectuée, préalablement à chaque utilisation, selon la procédure décrite dans le mode opératoire de l'appareil utilisé qui ici est un RHEOMAT 100.

### 3. Remplissage du godet

Remplir avec soin le godet, il faut éviter toute déstructuration du produit lors de son introduction dans le godet.

Le volume de substance à introduire dans chaque godet est le suivant :
Godet du mobile 1 : 320 mL mesuré ;
Godet du mobile 2 : jusqu'au repère soit 60 ml ;
Godet des mobiles 3, 4 et 5 : jusqu'au trop plein soit 25 ml.

### 4. Mesure

Placer sur l'appareil (RHEOMAT 100) le support du système et le corps de mesure sélectionné puis adapter le godet de mesure et mettre l'appareil en marche.

Lire la viscosité après 10 min de rotation du corps de mesure, la valeur donnée étant en UD et nécessite l'utilisation d'un abaque pour transposition en poises.

**Résultats :**

| | **Composition 1 (Témoin)** | **Composition 2 (Selon l'invention)** | **Composition 3 (comparatif)** |
|---|---|---|---|
| **Viscosité (25 °C)** | **570 cps** | **730 cps** | **570 cps** |

On observe une augmentation de la viscosité de la composition 2 comprenant le gel aqueux de phyllosilicate synthétique selon l'invention, ce qui n'est pas le cas pour la composition 3 comparative comprenant du talc naturel.

En conséquence, à l'inverse du gel aqueux de phyllosilicate synthétique selon l'invention, le talc n'impacte pas la viscosité d'une composition de type émulsion directe le comprenant. En revanche, un gel aqueux de phyllosilicate synthétique selon l'invention se comporte en un très bon épaississant de composition de type émulsion directe.

### 2.3. Evaluation visuelle du pouvoir homogénéisant après application des compositions 1 à 3

Les compositions 1 à 3 sont appliquées sur un dispositif d'évaluation conforme à ceux décrits dans la demande WO2014170807.

Ce dispositif comprend une surface d'application contenant différentes régions caractérisées par leur état de surface, qui traduit de préférence différents grades de typologies de peaux, telles que des peaux d'âge croissant, des peaux présentant une taille de pores croissante, des peaux présentant un nombre de défauts cutanés croissant et/ou des peaux présentant des rides de plus en plus marquées et/ou de plus en plus nombreuses.

Chacune des compositions 1 à 3 est appliquée au doigt, à raison de 2mg/cm², sur la surface d'application dédiée à recevoir l'échantillon à évaluer.

Le résultat obtenu a ensuite été évalué qualitativement en termes d'homogénéisation après application.

Les dépôts obtenus après application de ces compositions ont ainsi été observés à l'œil nu et au Microscope Electronique à Balayage (MEB) (4 ou 5 KV avec un grossissement x100), permettant ainsi d'avoir une vision globale.

Les résultats obtenus sont illustrés dans le tableau ci-après.

| | **Composition 1 (Témoin)** | **Composition 2 (Selon l'invention)** | **Composition 3 (comparatif)** |
|---|---|---|---|
| **Homogénéisation après application** | -- | ++ | -- |

### 2.4. Conclusion

Il est observé que la composition 2 selon l'invention, comprenant un gel aqueux ou hydroalcoolique de phyllosilicate synthétique selon l'invention, est significativement plus homogène après application qu'une composition comprenant du talc naturel, à l'image de la composition 3 comparative.

Plus particulièrement, la composition 1 témoin présente un dépôt poreux et fortement craquelé avec un marquage du relief.

La composition 3 comparative (i.e. contenant du talc naturel) présente également un dépôt poreux et craquelé avec une accentuation du marquage du relief.

Au contraire, la composition 2 selon l'invention (i.e. comprenant un gel aqueux de phyllosilicate synthétique selon l'invention) présente un dépôt significativement moins craquelé et le relief est fortement lissé.

### 2.5. Evaluation de l'effet correcteur des imperfections cutanées (soft-focus) des compositions 1 à 3

### 2.5.1. Conditions opératoires

Les propriétés optiques des compositions testées sont caractérisées à l'aide d'une mesure de Flou ou de Haze (effet voile ou calque) avec un appareil commercial de type « Hazemeter ». Le Haze correspond à la lumière transmise diffuse/lumière transmise totale. Les résultats sont exprimés en pourcentages. Plus la valeur de Haze est importante, plus l'effet soft focus (indice de flou) est élevé.

### Les mesures sont effectuées selon le protocole suivant :

a- étaler sur un film transparent plastique (Byk), une couche de 25,4 µm d'épaisseur humide de la composition dont on cherche à évaluer le Flou, à l'aide d'un étaleur automatique.
b- laisser sécher pendant 1 heure à température ambiante.
c- mesurer l'indice de flou (ou Haze) à l'aide d'un HAZE GARD de marque BYK GARDNER.

### 2.5.2. Résultats

### 2.6. Evaluation de l'effet matifiant des compositions 1 à 3

### Protocole de mesure de la matité d'une composition

La brillance d'un dépôt résultant de l'application d'une composition peut être communément mesurée selon diverses méthodes, telle que celle utilisant un Brillancemètre Byk Micro TRI gloss 20° / 60° / 85°.

### Principe de la mesure à l'aide de ce Brillancemètre

L'appareil éclaire l'échantillon à analyser selon une certaine incidence et mesure l'intensité de la réflexion spéculaire.

L'intensité de la lumière réfléchie dépend du matériau et de l'angle d'illumination. Pour des matériaux non ferreux (peinture, plastique), l'intensité de lumière réfléchie s'accroît avec l'angle d'illumination. Le reste de lumière incidente pénètre dans le matériau et selon la teinte de la couleur, il est soit absorbé en partie soit diffusé.

Les résultats de mesure du réflectomètre ne sont pas basés sur la quantité de lumière incidente mais sur un étalon en verre noir et poli d'indice de réfraction défini.

La mesure est normalisée par rapport à un étalon interne et ramenée à une valeur sur 100 : Pour cet étalon standard, la valeur de mesure est fixée à 100 unités de brillant (calibrage).

Plus la valeur mesurée est proche de 100 plus l'échantillon est brillant. L'unité de mesure est l'Unité de brillant (UB).

L'angle d'illumination utilisé influence grandement la valeur du réflectomètre. Pour pouvoir bien différencier des surfaces très brillantes et mates, la normalisation a défini 3 géométries, ou 3 domaines de mesure.

### Protocole de test

a- Sur une carte de contraste de marque LENETA et de référence FORM 1A PENOPAC, étaler une couche de 30 µm d'épaisseur humide de la composition dont on cherche à évaluer la brillance moyenne, à l'aide d'un étaleur automatique. La couche recouvre le fond blanc et le fond noir de la carte.
b- Laisser sécher pendant 24 heures à 37 °C.
c- Mesurer la brillance à 20°, à 60° et à 85° sur le fond absorbant mat blanc (3 mesures) a l'aide d'un Brillancemètre de marque BYK GARDNER et de référence microTRI-GLOSS.

Il convient ensuite de comparer les valeurs mesurées en UB obtenues pour les différentes compositions testées. Plus la valeur mesurée est faible, plus le dépôt est mat.

### Résultats

La composition 2 selon l'invention (i.e. comprenant un gel aqueux de phyllosilicate synthétique selon l'invention) présente un résultat mesuré en UB significativement inférieur à celui obtenu pour les autres compositions, et est par conséquent la composition conduisant au dépôt le plus mat.

### 2.7. Conclusions

Un gel aqueux de phyllosilicate synthétique selon l'invention permet avantageusement de conférer à une composition cosmétique de type émulsion directe le comprenant une viscosité plus élevée, de meilleures propriétés d'homogénéisation après application, ainsi qu'un effet matifiant, notamment comparée à une composition comprenant à titre de charge du talc naturel.

En outre, la présence d'un phyllosilicate synthétique permet d'améliorer l'effet correcteur des imperfections cutanées d'une composition de type émulsion directe le comprenant.

### EXEMPLE 3 : Influence de la nature de charge sur l'effet matifiant et homogénéisant après application d'une composition cosmétique de type émulsion inverse eau-dans-huile

### 3.1. Compositions testées

Les matières de la phase A sont tout d'abord pesées puis mises sous agitation Moritz à 68 °C. Les pigments sont ensuite broyés dans l'huile de la phase B puis ajoutés à la phase A.

La phase C est alors ajoutée et le mélange ainsi obtenu est laissé sous agitation jusqu'à homogénéisation.

Les matières de la phase D sont ensuite pesées, mélangées et laissées homogénéiser sous agitation à l'aide d'un barreau aimanté. La phase D est ensuite introduite dans la phase A, avant d'émulsionner le mélange sous vive agitation pendante 10 minutes.

On laisse enfin refroidir à 30 °C puis la phase E est introduite.

La tare en eau est ensuite faite.

Les compositions suivantes ont alors été préparées.

Les pourcentages indiqués dans ce tableau sont des pourcentages en poids du poids total de la composition correspondante.

| | **Matières premières (Références commerciales)** | **Composition 4 (Témoin)** | **Composition 5 (Selon l'invention)** | **Composition 6 (Comparatif)** |
|---|---|---|---|---|
| **A** | PEG-10 diméthicone (KF-6017® de SHIN ETSU) | 2,00 % | 2,00 % | 2,00 % |
| | Cétyl PEG/PPG-10/1 Diméthicone (ABIL EM 90® de EVONIK GOLDSCHMIDT) | 1,00 % | 1,00 % | 1,00 % |
| | Cyclohexasiloxane (KF-996® de SHIN ETSU) | 8,20 % | 8,20 % | 8,20 % |
| | Isododécane | 1,00 % | 1,00 % | 1,00 % |
| | Isohexadécane | 1,60 % | 1,60% | 1,60 % |
| | Ethylhexyl méthoxycinnamate | 3,00 % | 3,00 % | 3,00 % |
| | Copolymère acrylates/ polytriméthyl siloxyméthacrylate (DOW CORNING FA 4002 ID SILICONE ACRYLATE® de DOW CORNING) | 10,0 % | 10,0 % | 10,0 % |
| **B** | Pigments NAI (SUNPURO) | 12,0 % | 12,0 % | 12,0 % |
| | Cyclohexasiloxane | 7,50 % | 7,50 % | 7,50 % |
| **C** | Silice | 1,00 % | 1,00 % | 1,00 % |
| | Nylon-12 | 3,00 % | 3,00 % | 3,00 % |
| | Perlite | 0,20 % | 0,20 % | 0,20 % |
| **D** | Eau | 34,80 % | 16,80 % | 33,33 % |
| | Gel de phyllosilicate synthétique selon l'invention (**8,20%** ma) dans eau | **0,00 %** | **18,00 % (soit 1,47% MA*)** | **0,00 %** |
| | Butylène glycol | 6,00 % | 6,00 % | 6,00 % |
| | Sulfate de magnésium | 0,70 % | 0,70 % | 0,70 % |
| | **Talc naturel** (LUZENAC PHARMA M) | **0,00 %** | **0,00 %** | **1,47 %** |
| **E** | Alcool dénaturé | 8% | 8% | 8% |
| **TOTAL** | | 100 | 100 | 100 |

| | | | | |
|---|---|---|---|---|
| *MA : Matière Active | | | | |

### 3.2. Evaluation de la viscosité des compositions 4 à 6

Le protocole de mesure de la viscosité est identique à celui détaillé en exemple 2 ci-dessus.

**Résultats :**

| | **Composition 4 (Témoin)** | **Composition 5 (Selon l'invention)** | **Composition 6 (comparatif)** |
|---|---|---|---|
| **Viscosité (25 °C)** | 9 poises | 12 poises | 9 poises |

### 3.3. Evaluation visuelle du pouvoir homogénéisant des compositions 4 à 6

Les compositions 4 à 6 sont testées conformément à ce qui est indiqué en exemple 2 ci-dessus.

Les résultats obtenus sont illustrés dans le Tableau ci-après.

| | **Composition 4 (Témoin)** | **Composition 5 (selon l'invention)** | **Composition 6 (comparatif)** |
|---|---|---|---|
| **Homogénéisation après application** | -/+ | +/+ | -/+ |

### 3.4. Evaluation de l'effet matifiant des compositions 4 à 6

Le protocole de mesure de la matité est identique à celui décrit en exemple 2 ci-dessus (voir item 2.6).

### Résultats

La composition 5 selon l'invention (i.e. comprenant un gel aqueux de phyllosilicate synthétique selon l'invention) présente un résultat mesuré en UB inférieur à celui obtenu pour les autres compositions, et est par conséquent la composition conduisant au dépôt le plus mat.

### 3.5. Conclusions

On observe une légère augmentation de la viscosité de la composition 5 selon l'invention (i.e. comprenant un gel aqueux de phyllosilicate synthétique selon l'invention), ce qui n'est pas le cas pour la composition 6 comparative comprenant du talc naturel.

En d'autres termes, à l'inverse d'un gel aqueux de phyllosilicate synthétique selon l'invention, le talc naturel n'impacte pas la viscosité d'une composition de type émulsion inverse le comprenant. En revanche, un gel aqueux de phyllosilicate synthétique selon l'invention se comporte en un très bon épaississant de composition de type émulsion inverse.

En outre, la composition 5 contenant le gel aqueux de phyllosilicate synthétique selon l'invention est significativement plus homogène après application que la composition 6 comparative comprenant du talc naturel.

Enfin, la présence d'un gel aqueux de phyllosilicate synthétique selon l'invention permet de renforcer la matité d'une composition de type émulsion inverse le comprenant et la tenue de cet effet matifiant dans le temps.

### EXEMPLE 4 : Influence de la nature de charge sur l'effet matifiant et homogénéisant après application d'une composition cosmétique de type gel-gel

### 4.1. Compositions testées

Toutes les matières premières sont pesées dans un bécher puis mises sous vive agitation au rayneri jusqu'à ce que le mélange devienne homogène.

Les compositions 7 à 8 suivantes ont alors été préparées.

Les pourcentages indiqués dans ce tableau sont des pourcentages en poids du poids total de la composition correspondante.

| **Matières premières (références commerciales)** | **Composition 7 (Témoin)** | **Composition 8 (Selon l'invention)** | **Composition 9 (Comparatif)** |
|---|---|---|---|
| Eau désionisée micro biologiquement propre | 51,54 % | 33,54 % | 49,54 % |
| Glycérine | 5,28 % | 5,28 % | 5,28 % |
| Copolymère acrylamido-2-méthyl propane sulfonate de sodium / hydroxyéthylacrylate sous forme de poudre (SEPINOV EMT 10® de SEPPIC) | 2,4 % | 2,4 % | 3,17% |
| Phénoxv-2 éthanol | 0,78 % | 0,78 % | 0,78 % |
| Gel de phyllosilicate synthétique selon l'invention à 11,0% dans eau | **0,00 %** | **18,00 % (soit 2,00% MA*)** | **0,00 %** |
| Talc naturel (LUZENAC PHARMA M) | 0,00% | 0,00 % | 2,00 % |
| Poly diméthylsiloxane (viscosité: 5 CST) (XIAMETER PMX-200 SILICONE FLUID 5CS® de DOW CORNING) | 8,00 % | 8,00 % | 8,00 % |
| Mélange de polydiméthylsiloxane réticulé par l'hexadiène / poly diméthylsiloxane 5cst (DOW CORNING 9041 SILICONE ELASTOMER BLEND® de DOW CORNING) | 14,0% | 14,0 % | 14,0 % |
| Pigments NAI (SUNPURO) | 18,0 % | 18,0 % | 18,0 % |
| **TOTAL** | 100 | 100 | 100 |

| | | | |
|---|---|---|---|
| *MA : Matière Active | | | |

### 4.2. Evaluation de la viscosité des compositions 7 à 9

Le protocole de mesure de la viscosité est identique à celui détaillé en exemple 2 ci-dessus.

**Résultats :**

| | **Composition 7 (Témoin)** | **Composition 8 (Selon l'invention)** | **Composition 9 (comparatif)** |
|---|---|---|---|
| **Viscosité (25 °C)** | 64 poises | **123 poises** | 64 poises |

### 4.3. Evaluation de l'effet matifiant des compositions 7 à 9

Le protocole de mesure de la matité est identique à celui décrit en exemple 2 ci-dessus.

### Résultats

La composition 8 selon l'invention (i.e. comprenant un gel aqueux de phyllosilicate synthétique selon l'invention) présente un résultat mesuré en UB inférieur à celui obtenu pour les autres compositions, et est par conséquent la composition conduisant au dépôt le plus mat.

### 4.4. Conclusions

On observe une augmentation significative de la viscosité de la composition 8 selon l'invention (i.e. comprenant un gel aqueux de phyllosilicate synthétique selon l'invention), ce qui n'est pas le cas pour la composition 9 comparative comprenant du talc naturel.

En d'autres termes, à l'inverse d'un gel aqueux de phyllosilicate synthétique selon l'invention, le talc n'impacte pas la viscosité d'une composition de type gel-gel le comprenant. En revanche, un gel aqueux de phyllosilicate synthétique selon l'invention se comporte en un très bon épaississant d'une composition de type gel-gel.

En outre, la présence d'un gel aqueux de phyllosilicate synthétique selon l'invention permet de renforcer la matité d'une composition de type gel-gel le comprenant et la tenue de cet effet matifiant dans le temps.

### EXEMPLE 5 : Influence du gel de phyllosilicate comme agent gélifiant et matifiant d'une composition cosmétique de soin

Les pourcentages indiqués dans ce tableau sont des pourcentages en poids du poids total de la composition correspondante.

### 5.1. Compositions testées

| Type Cosmétique | Noms INCI | Composition 10 (Témoin) | Composition 11 (Selon l'invention) | Composition 12 (Comparatif) |
|---|---|---|---|---|
| PHASE GRASSE | Alcool béhénique (et) glycéryl stéarate (et) disodium ethylène dicocamide PEG-15 disulfate (et) Glycéryl stéarate citrate (CERULATION H® de Sasol) | 3,00 % | 3,00 % | 3,00 % |
| | Isostéaryl néopentanoate (DUB VCI 18® STEARINERIE DUBOIS) | 2,50% | 2,50% | 2,50% |
| | Isohexadécane (Isohexadécane® INEOS) | 8,00 % | 8,00 % | 8,00 % |
| | Hexyldécyle Laurate (et) Hexyldécanole (CETIOL PGL COGNIS® (BASF)) | 4,00 % | 4,00 % | 4,00 % |
| | Polyalkylacrylate en C₁₀-C₃₀ (INTELIMER IPA 13-1® NG POLYMERAIR PRODUCTS AND CHEMICALS) | 0,50 % | 0,50 % | 0,50% |
| PHASE AQUEUSE | Gel de phyllosilicate synthétique conforme à l'invention (10,3% ma dans de l'eau) | **0,00 %** | **36 % MP (soit 3,7 % ma)** | **0,00 %** |
| | Eau | QSP 100 | QSP 100 | QSP 100 |
| | Glycérine | 7,00 % | 7,00 % | 7,00 % |
| | Conservateur | QS | QS | QS |
| | Ammonium Polyacryloyldimethyl Taurate (HOSTACERIN AMPS® CLARIANT) | **0,00 %** | **0,00 %** | **0,50 %** |
| | Gomme de Xanthane (RHODICARE XC® RHODIA (SOLVAY)) | 0,20 % | 0,20 % | 0,20 % |

### 5.2. Evaluation de la viscosité des compositions 10 à 12

Le protocole de mesure de la viscosité est identique à celui détaillé en exemple 2 ci-dessus.

**Résultats :**

| | **Composition 10 (Témoin)** | **Composition 11 (Selon l'invention)** | **Composition 12 (comparatif)** |
|---|---|---|---|
| **Viscosité (25 °C)** | < 1 poise | **12,2 poises** | 11,5 poises |

### 5.3. Evaluation de l'effet matifiant des compositions 10 à 12

Le protocole de mesure de la matité est identique à celui décrit en exemple 2 ci-dessus.

**Résultats**

| | Composition 10 (Témoin) | Composition 11 (Selon l'invention) | Composition 12 (comparatif) |
|---|---|---|---|
| Brillance 60° | ++++ | + | ++++++ |

La composition 11 selon l'invention (i.e. comprenant un gel aqueux de phyllosilicate synthétique selon l'invention) présente un résultat mesuré en UB très significativement inférieur à celui obtenu pour les autres compositions, et est par conséquent la composition conduisant au dépôt le plus mat.

### 5.4. Conclusions

On observe une augmentation significative de la viscosité de la composition 11 selon l'invention (i.e. comprenant un gel aqueux de phyllosilicate synthétique selon l'invention), ce qui n'est pas le cas pour la composition 12 comparative comprenant, à la place du gel selon l'invention, un gélifiant non conforme à l'invention.

En outre, la présence d'un gel aqueux de phyllosilicate synthétique selon l'invention permet de renforcer très significativement la matité et la tenue de cet effet matifiant dans le temps d'une composition le comprenant, comparativement à une composition exempte d'un tel gel.

### EXEMPLE 6 : Influence de la nature de charge sur les propriétés d'étalement d'une composition cosmétique aqueuse

Les pourcentages indiqués dans ce tableau sont des pourcentages en poids du poids total de la composition correspondante.

### 6.1. Compositions testées

| REFERENCE COMMERCIALE (fournisseur) | **Composition 13 (Selon l'invention)** | **Composition 14 (Comparatif)** | **Composition 15 (Solution aqueuse de hyaluronate de Na) (Comparatif)** |
|---|---|---|---|
| Eau | Qsp100 | Qsp 100 | Qsp 100 |
| Hyaluronate de sodium | 1 % | 1% | 1 % |
| Gel de phyllosilicate synthétique selon l'invention (11% ma) | **18,18 % (soit 2% ma)** | 0 % | 0% |
| Talc naturel (LUZENAC ® PHARMA M) | 0% | **2 %** | 0 % |
| Total | 100 | 100 | 100 |

### 6.2. Exemple de l'invention comparativement au contre-exemple 14 :

Le talc naturel ne se disperse pas dans la solution aqueuse de la composition 14 et reste en surface. Le gel de phyllosilicate synthétique selon l'invention permet au contraire l'obtention d'un gel macroscopiquement homogène.

### 6.3. Homogénéité du dépôt après application

La nacre de référence « NACRE COLORONA KARAT GOLD MP-24 » est utilisée en tant que traceur pour vérifier visuellement l'homogénéité du dépôt après application.

Ainsi, 2 grammes de cette nacre sont additionnés à 100 grammes de la composition 13 selon l'invention et à la composition comparative 15.

Il apparait que le dépôt obtenu après application de la composition 13 selon l'invention est visuellement plus homogène sur la peau contrairement au dépôt obtenu avec la composition comparative 15 ne comprenant pas le gel de phyllosilicate synthétique selon l'invention.

### 6.4. Matité

Le protocole de mesure de la matité est identique à celui décrit en exemple 2 ci-dessus.

La composition 13 selon l'invention (i.e. comprenant un gel aqueux de phyllosilicate synthétique selon l'invention) présente un résultat mesuré en UB très significativement inférieur à celui obtenu pour les autres compositions, et est par conséquent la composition conduisant au dépôt le plus mat.

## Revendications

1. Procédé de préparation d'une composition cosmétique, dans lequel un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å est incorporé dans la phase aqueuse de ladite composition.

2. Utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent viscosant dans une composition cosmétique, ledit gel aqueux ou hydroalcoolique de phyllosilicate synthétique étant incorporé dans la phase aqueuse de ladite composition.

3. Utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent matifiant dans une composition cosmétique, ledit gel aqueux ou hydroalcoolique de phyllosilicate synthétique étant incorporé dans la phase aqueuse de ladite composition.

4. Utilisation d'un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, à titre d'agent homogénéisant d'application dans une composition cosmétique, ledit gel aqueux ou hydroalcoolique de phyllosilicate synthétique étant incorporé dans la phase aqueuse de ladite composition.

5. Composition, notamment cosmétique, comprenant au moins un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å et au moins un ingrédient additionnel, distinct dudit gel de phyllosilicate synthétique, choisi parmi les corps gras siliconés tels que les huiles, les gommes et les cires de silicone; les corps gras non siliconés tels que les huiles, les pâteux et les cires d'origine végétale, minérale, animale et/ou synthétique; les acides gras ayant de 8 à 32 atomes de carbone; les esters et les éthers de synthèse; les hydrocarbures linéaires ou ramifiés, d'origine minérale ou synthétique; les alcools gras ayant de 8 à 26 atomes de carbone; les alcools en C₂-C₆; les glycols; les tensioactifs ; les gélifiants aqueux ou huileux ; les actifs cosmétiques ; les parfums ; les charges ; les matières colorantes ; les vitamines ; les conservateurs ; les polymères filmogènes ; et leurs mélanges, ladite composition contenant une phase aqueuse formée au moins en partie par ledit gel aqueux ou hydroalcoolique de phyllosilicate synthétique.

6. Composition selon la revendication 5, **caractérisée en ce qu'**elle présente une bande d'absorption infrarouge à 7200 cm⁻¹, correspondant à la vibration d'élongation attribuée aux groupes silanols Si-OH en bordure des feuillets du phyllosilicate.

7. Composition selon l'une quelconque des revendications 5 ou 6, **caractérisée par** une absence de bande d'absorption à 7 156 cm⁻¹.

8. Composition selon l'une quelconque des revendications 5 à 7, **caractérisée en ce que** le gel aqueux ou hydroalcoolique de phyllosilicate synthétique constitue la phase aqueuse.

9. Composition selon l'une quelconque des revendications 5 à 8, **caractérisée en ce que** le gel aqueux ou hydroalcoolique de phyllosilicate synthétique est présent dans la composition en une teneur allant de 0,01 % à 40 % en poids en matière active, en particulier allant de 0,05 % à 30 % en poids en matière active, de préférence allant de 0,1 % à 20 % en poids en matière active, et plus préférentiellement de 0,2 % à 10 % en poids en matière active, par rapport au poids total de la composition.

10. Composition selon l'une quelconque des revendications 5 à 9, ledit ingrédient additionnel étant au moins un composé constitutif d'une phase aqueuse, de préférence choisi parmi les monoalcools inférieurs ayant de 1 à 5 atomes de carbone et les polyols, notamment les polyols présentant de 2 à 32 atomes de carbone.

11. Composition selon l'une quelconque des revendications 5 à 10, ledit ingrédient additionnel étant au moins un composé constitutif d'une phase grasse, et en particulier choisi parmi une huile hydrocarbonée ou siliconée, polaire ou apolaire, volatile ou non volatile, une cire, un composé pâteux, et un de leurs mélanges, de préférence choisi parmi au moins une huile siliconée volatile ou non volatile ou parmi les huiles hydrocarbonées volatiles en C₈-C₁₆, les éthers de synthèse ayant de 10 à 40 atomes de carbone, les esters de synthèse, les esters de polyols et les esters du pentaérythritol, les alcools gras liquides à température ambiante à chaîne carbonée ramifiée et/ou insaturée ayant de 8 à 26 atomes de carbone, les acides gras supérieurs en C₁₂-C₂₂; et leurs mélanges.

12. Composition selon l'une quelconque des revendications 5 à 11, ledit ingrédient additionnel étant au moins un composé choisi parmi les tensioactifs non ioniques, anioniques ou amphotères ; les charges ; les agents gélifiants ; et leurs mélanges.

13. Composition selon l'une quelconque des revendications 5 à 12, ledit agent gélifiant étant :
- un gélifiant hydrophile choisi parmi les silices pyrogénées, les gélifiants polymériques synthétiques, les gélifiants polymériques naturels ou d'origine naturelle, les homo- ou copolymères d'acides acrylique ou méthacrylique, leurs sels ou leurs esters, les copolymères acide polyacryliques/acrylates d'alkyle, les copolymères AMPS/méthacrylates d'alkyle polyoxyéthylénés, réticulés ou non réticulés, les polymères de chitine ou de chitosane anioniques, cationiques, amphotères ou non-ioniques, les polymères de cellulose, distincts de l'alkylcellulose, choisis parmi l'hydroxyéthylcellulose, l'hydroxypropylcellulose, l'hydroxyméthylcellulose, l'éthylhydroxyéthylcellulose, la carboxyméthylcellulose, ainsi que les dérivés quatemisés de la cellulose, les polymères vinyliques et les polymères associatifs anioniques, cationiques, non-ioniques ou amphotères, les polymères d'origine naturelle modifiés ou non modifiés, les alginates et les carraghénanes, les glycoaminoglycanes, l'acide désoxyribonucléique, les muccopolysaccharides en particulier l'acide hyaluronique et ses dérivés, les chondroïtines sulfate, et leurs mélanges ;
- un gélifiant lipophile choisi parmi les gélifiants particulaires, les élastomères d'organopolysiloxane, les polymères semi-cristallins, les copolymères séquencés hydrocarbonés, les esters de dextrine, les polymères à liaison hydrogène et leurs mélanges ;
et leurs mélanges.

14. Composition selon l'une quelconque des revendications 5 à 13, ledit ingrédient additionnel étant au moins un composé choisi parmi les actifs cosmétiques ; les vitamines ; les filtres anti-UV ; les matières colorantes, de préférence choisies parmi les pigments et les nacres ; les parfums ; et leurs mélanges.

15. Composition selon l'une quelconque des revendications 5 à 14, **caractérisée en ce que** ladite composition se présente sous la forme d'une composition de soin et/ou de maquillage de la peau et/ou des ongles, du corps ou du visage, en particulier du visage, et est de préférence un fond de teint, un fard à joues ou à paupières.

16. Composition selon l'une quelconque des revendications 5 à 15, **caractérisée en ce que** ladite composition est dédiée au soin et/ou maquillage des lèvres et est de préférence un stick, un vernis ou un gloss.

17. Procédé de maquillage et/ou de soin de la peau et/ou des ongles comprenant au moins une étape d'application sur ladite peau et/ou lesdits ongles d'une composition comprenant, dans un milieu physiologiquement acceptable, au moins un gel aqueux ou hydroalcoolique de phyllosilicate synthétique de formule Mg₃Si₄O₁₀(OH)₂ présentant une raie de diffraction des rayons X supérieure à 9,4 Å et inférieure ou égale à 9,8 Å, ladite composition comprenant une phase aqueuse formée au moins en partie par ledit gel aqueux ou hydroalcoolique de phyllosilicate synthétique.

18. Procédé selon la revendication précédente dans lequel ladite composition est telle que définie en revendications 5 à 16.

19. Procédé selon la revendication 17 ou 18, **caractérisé en ce que** ledit procédé est dédié à procurer un résultat maquillage matifiant et/ou homogène du teint de la peau après application.

20. Procédé selon la revendication 17 ou 18, **caractérisé en ce que** ledit procédé est dédié à faciliter l'étalement sur la peau de la composition et à conduire à un dépôt homogène d'actifs sur la peau après application, notamment choisis parmi les actifs cosmétiques ; les vitamines ; les filtres anti-UV ; les matières colorantes, de préférence choisies parmi les pigments et les nacres ; les parfums ; et leurs mélanges.

## Patentansprüche

1. Verfahren zur Herstellung einer kosmetischen Zusammensetzung, wobei ein wässriges oder hydroalkoholisches Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, in der wässrigen Phase dieser Zusammensetzung integriert ist.

2. Verwendung eines wässrigen oder hydroalkoholischen Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, als Viskositätsmittel in einer kosmetischen Zusammensetzung, wobei das wässrige oder hydroalkoholische Gel aus synthetischem Phyllosilikat in die wässrige Phase der Zusammensetzung integriert ist.

3. Verwendung eines wässrigen oder hydroalkoholischen Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, als Mattierungsmittel in einer kosmetischen Zusammensetzung, wobei das wässrige oder hydroalkoholische Gel aus synthetischem Phyllosilikat in die wässrige Phase der Zusammensetzung integriert ist.

4. Verwendung eines wässrigen oder hydroalkoholischen Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, als Homogenisierungsmittel in einer kosmetischen Zusammensetzung, wobei das wässrige oder hydroalkoholische Gel aus synthetischem Phyllosilikat in die wässrige Phase der Zusammensetzung integriert ist.

5. Zusammensetzung, insbesondere kosmetische Zusammensetzung, umfassend mindestens ein wässrigen oder hydroalkoholischen Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, und mindestens einen zusätzlichen Bestandteil, der sich von dem Gel aus synthetischen Phyllosilikat unterscheidet, ausgewählt aus den Silikonfetten, wie z. B. Silikonöle, -gummis und -wachse; silikonfreien Fetten, wie z. B. Öle, Pasten und Wachse pflanzlichen, mineralischen, tierischen und/oder synthetischen Ursprungs; Fettsäuren mit 8 bis 32 Kohlenstoffatomen; synthetischen Estern und Ethern; linearen oder verzweigten Kohlenwasserstoffen mineralischen oder synthetischen Ursprungs; Fettalkoholen mit 8 bis 26 Kohlenstoffatomen; C₂-C₆-Alkoholen; Glykolen; grenzflächenaktiven Stoffen; wässrigen oder öligen Geliermitteln; kosmetischen Wirkstoffen; Parfüms; Füllstoffen; Farbstoffen; Vitaminen; Konservierungsmitteln; filmbildenden Polymeren; und ihren Gemischen, wobei die Zusammensetzung eine wässrige Phase enthält, die mindestens teilweise durch das wässrige oder hydroalkoholische Gel aus synthetischem Phyllosilikat gebildet ist.

6. Zusammensetzung nach Anspruch 5, **dadurch gekennzeichnet, dass** sie eine Infrarot-Absorptionsband bei 7200 cm⁻¹ entsprechend der Streckschwingung, die den Si-OH-Silanolgruppen am Rand der Phyllosilikatplatten zugeschrieben wird.

7. Zusammensetzung nach einem der Ansprüche 5 oder 6, **gekennzeichnet durch** ein Fehlen eines Absorptionsbands bei 7156 cm⁻¹.

8. Zusammensetzung nach einem der Ansprüche 5 bis 7, **dadurch gekennzeichnet, dass** das wässrige oder hydroalkoholische Gel aus synthetischem Phyllosilikat die wässrige Phase bildet.

9. Zusammensetzung nach einem der Ansprüche 5 bis 8, **dadurch gekennzeichnet, dass** das wässrige oder hydroalkoholische Gel aus synthetischem Phyllosilikat in der Zusammensetzung in einem Gehalt von 0,01 bis 40 Gewichtsprozent Wirkstoff, insbesondere von 0,05 bis 30 Gewichtsprozent Wirkstoff, vorzugsweise von 0,1 bis 20 Gewichtsprozent Wirkstoff und bevorzugter von 0,2 bis 10 Gewichtsprozent Wirkstoff, bezogen auf das Gesamtgewicht der Zusammensetzung, vorhanden ist.

10. Zusammensetzung nach einem der Ansprüche 5 bis 9, wobei der zusätzliche Bestandteil mindestens eine Verbindung ist, die eine wässrige Phase bildet, vorzugsweise ausgewählt aus den niedrigen einwertigen Alkoholen mit 1 bis 5 Kohlenstoffatomen und den Polyolen, insbesondere Polyole mit 2 bis 32 Kohlenstoffatomen.

11. Zusammensetzung nach einem der Ansprüche 5 bis 10, wobei der zusätzliche Bestandteil mindestens eine Verbindung ist, die eine Fettphase bildet und insbesondere ausgewählt ist aus einem polaren oder apolaren, flüchtigen oder nicht flüchtigen, Kohlenwasserstoff- oder Silikonöl, einem Wachs, einer pastösen Verbindung und einem ihrer Gemische, vorzugsweise ausgewählt aus mindestens einem flüchtigen oder nicht flüchtigen Silikonöl oder aus flüchtigen C₈-C₁₆-Kohlenwasserstoffölen, synthetischen Ethern mit 10 bis 40 Kohlenstoffatomen, synthetischen Estern, Polyolestern und Pentaerythritolestern, bei Raumtemperatur flüssigen Fettalkoholen mit einer verzweigten und/oder ungesättigten Kohlenstoffkette mit 8 bis 26 Kohlenstoffatomen, höheren C₁₂-C₂₂-Fettsäuren und ihren Gemischen davon.

12. Zusammensetzung nach einem der Ansprüche 5 bis 11, wobei der zusätzliche Bestandteil mindestens eine Verbindung ist, ausgewählt aus nichtionischen, anionischen oder amphoteren Tensiden; Füllstoffen, Geliermitteln; und Gemischen davon.

13. Zusammensetzung nach einem der Ansprüche 5 bis 12, wobei das Geliermittel wie folgt ist:
- ein hydrophiles Geliermittel, ausgewählt aus pyrogenen Kieselsäuren, synthetischen polymeren Geliermitteln, natürlichen polymeren Geliermitteln oder Geliermitteln natürlichen Ursprungs, Homo- oder Copolymeren von Acryl- oder Methacrylsäure, ihren Salzen oder Estern, Polyacrylsäure/Alkylacrylat-Copolymeren, vernetzten oder unvernetzten AMPS/polyoxyethylenierten Alkylmethacrylat-Copolymeren, anionischen, kationischen, amphoteren oder nichtionischen Chitin- oder Chitosan-Polymeren, von Alkylcellulose verschiedenen Cellulosepolymeren, ausgewählt aus Hydroxyethylcellulose, Hydroxypropylcellulose, Hydroxymethylcellulose, Ethylhydroxyethylcellulose, Carboxymethylcellulose sowie quaternisierten Derivaten von Cellulose, Vinylpolymeren und anionischen, kationischen, nichtionischen oder amphoteren assoziativen Polymeren, modifizierten oder unmodifizierten Polymeren natürlichen Ursprungs, Alginaten und Carrageenen, Glycoaminen, Desoxyribonukleinsäure, Mukopolysaccharide, insbesondere Hyaluronsäure und ihren Derivaten, Chondroitinsulfat und Gemischen davon;
- ein lipophiles Geliermittel, ausgewählt aus teilchenförmigen Geliermitteln, Organopolysiloxanelastomeren, halbkristallinen Polymeren, Kohlenwasserstoff-Blockcopolymeren, Dextrinestern, wasserstoffgebundenen Polymeren und Gemischen davon;
und Gemische davon.

14. Zusammensetzung nach einem der Ansprüche 5 bis 13, wobei der zusätzliche Bestandteil mindestens eine Verbindung ist, ausgewählt aus kosmetischen Wirkstoffen; Vitaminen; UV-Filtern; Farbmitteln, vorzugsweise ausgewählt aus Pigmenten und Perlglanzmaterialien; Parfüms; und Gemischen davon.

15. Zusammensetzung nach einem der Ansprüche 5 bis 14, **dadurch gekennzeichnet, dass** die Zusammensetzung in Form einer Zusammensetzung zur Pflege und/oder zum Schminken der Haut und/oder der Nägel, des Körpers oder des Gesichts, insbesondere des Gesichts, vorliegt und vorzugsweise ein Make-up, ein Rouge oder ein Lidschatten ist.

16. Zusammensetzung nach einem der Ansprüche 5 bis 15, **dadurch gekennzeichnet, dass** die Zusammensetzung zur Pflege und/oder zum Schminken der Lippen bestimmt ist und vorzugsweise ein Stift, ein Lack oder ein Gloss ist.

17. Verfahren zum Schminken und/oder zur Pflege der Haut und/oder der Nägel, mindestens umfassend einen Schritt eines Auftragens einer Zusammensetzung auf die Haut und/oder die Nägel, die in einem physiologisch annehmbaren Medium mindestens ein wässriges oder hydroalkoholisches Gel aus synthetischem Phyllosilikat der Formel Mg₃Si₄O₁₀(OH)₂, das ein Röntgenbeugungsmuster größer als 9,4 Å und kleiner als oder gleich wie 9,8 Å aufweist, wobei die Zusammensetzung eine wässrige Phase umfasst, die mindestens teilweise aus dem wässrigen oder hydroalkoholischen Gel aus synthetischem Phyllosilikat gebildet ist.

18. Verfahren nach dem vorvorherigen Anspruch, wobei die Zusammensetzung wie definiert in den Ansprüchen 5 bis 16 ist.

19. Verfahren nach Anspruch 17 oder 18, **dadurch gekennzeichnet, dass** das Verfahren dazu bestimmt ist, nach dem Auftragen ein mattierendes und/oder homogenes Make-up-Ergebnis für den Hautton zu erzielen.

20. Verfahren nach Anspruch 17 oder 18, **dadurch gekennzeichnet, dass** das Verfahren dazu bestimmt ist, die Verteilung der Zusammensetzung auf der Haut zu erleichtern und nach Auftragen zu einer homogenen Ablagerung von Wirkstoffen auf der Haut zu führen, die insbesondere ausgewählt sind aus kosmetischen Wirkstoffen; Vitaminen; UV-Filtern; Farbstoffen, vorzugsweise ausgewählt aus Pigmenten und Perlmutt; Parfüms; und Gemischen davon.

## Claims

1. Process for preparing a cosmetic composition wherein an aqueous or aqueous-alcoholic gel of synthetic phyllosilicate of formula Mg₃Si₄O₁₀(OH)₂ having an X-ray diffraction line greater than 9.4 Å and less than or equal to 9.8 Å. is incorporated in the aqueous phase of said composition.

2. The use of an aqueous or aqueous-alcoholic gel of synthetic phyllosilicate of formula Mg₃Si₄O₁₀(OH)₂ having an X-ray diffraction line greater than 9.4 Å and less than or equal to 9.8 Å as viscosity-enhancing agent in a cosmetic composition, said aqueous or aqueous-alcoholic gel of synthetic phyllosilicate being incorporated in the aqueous phase of said composition.

3. The use of an aqueous or aqueous-alcoholic gel of synthetic phyllosilicate having an X-ray diffraction line greater than 9.4 Å and less than or equal to 9.8 Å as matt-effect agent in a cosmetic composition, said aqueous or aqueous-alcoholic gel of synthetic phyllosilicate being incorporated in the aqueous phase of said composition.

4. The use of an aqueous or aqueous-alcoholic gel of synthetic phyllosilicate of formula Mg₃Si₄O₁₀(OH)₂ having an X-ray diffraction line greater than 9.4 Å and less than or equal to 9.8 Å. as application-homogenizing agent in a cosmetic composition, said aqueous or aqueous-alcoholic gel of synthetic phyllosilicate being incorporated in the aqueous phase of said composition.

5. Composition, in particular a cosmetic composition, comprising at least one aqueous or aqueous-alcoholic gel of synthetic phyllosilicate of formula Mg₃Si₄O₁₀(OH)₂ having an X-ray diffraction line greater than 9.4 Å and less than or equal to 9.8 Å, and at least one additional ingredient different from said synthetic phyllosilicate gel, chosen from silicone fatty substances such as silicone oils, gums and waxes; non-silicone fatty substances such as oils, pasty substances and waxes of plant, mineral, animal and/or synthetic origin; fatty acids containing from 8 to 32 carbon atoms; synthetic esters and ethers; linear or branched hydrocarbons of mineral or synthetic origin; fatty alcohols containing from 8 to 26 carbon atoms; C₂-C₆ alcohols; glycols; surfactants; aqueous or oily gelling agents; cosmetic active agents; fragrances; fillers; dyestuffs; vitamins; preserving agents; film-forming polymers; and mixtures thereof, said composition containing an aqueous phase formed at least in part by said aqueous or aqueous-alcoholic gel of synthetic phyllosilicate

6. The composition as claimed in claim 5, **characterized in that** it has an infrared absorption band at 7200 cm⁻¹, corresponding to the stretching vibration attributed to the silanol groups Si-OH at the edge of the phyllosilicate sheets.

7. The composition as claimed in any one of claims 5 or 6, **characterized by** an absence of absorption band at 7156 cm⁻¹.

8. The composition as claimed in any one of claims 5 to 7, **characterized in that** the aqueous or aqueous-alcoholic gel of synthetic phyllosilicate constitutes the aqueous phase.

9. The composition as claimed in any one of claims 5 to 8, **characterized in that** the aqueous or aqueous-alcoholic gel of synthetic phyllosilicate is present in the composition in a content ranging from 0.01% to 40% by weight of active material, in particular ranging from 0.05% to 30% by weight of active material, preferably ranging from 0.1% to 20% by weight of active material and more preferentially from 0.2% to 10% by weight of active material, relative to the total weight of the composition.

10. The composition as claimed in any one of claims 5 to 9, said additional ingredient being at least one constituent compound of an aqueous phase, preferably chosen from lower monoalcohols containing from 1 to 5 carbon atoms and polyols, especially polyols containing from 2 to 32 carbon atoms.

11. The composition as claimed in any one of claims 5 to 10, said additional ingredient being at least one constituent compound of a fatty phase, and in particular chosen from a polar or apolar, volatile or non-volatile hydrocarbon- or silicone-based oil, a wax, a pasty compound, and a mixture thereof, preferably chosen from among at least one volatile or non-volatile silicone-based oil or from among volatile C₈-C₁₆ hydrocarbon-based oils, synthetic ethers containing from 10 to 40 carbon atoms, synthetic esters, polyol esters and pentaerythritol esters, fatty alcohols that are liquid at room temperature bearing a branched and/or unsaturated carbon chain containing from 8 to 26 carbon atoms, C₁₂-C₂₂ higher fatty acids; and mixtures thereof.

12. The composition as claimed in any one of claims 5 to 11, said additional ingredient being at least one compound chosen from nonionic, anionic or amphoteric surfactants; fillers; gelling agents; and mixtures thereof.

13. The composition as claimed in any one of claims 5 to 12, said gelling agent being:
- a hydrophilic gelling agent chosen from fumed silicas, synthetic polymeric gelling agents, polymeric gelling agents that are natural or of natural origin, acrylic or methacrylic acid homopolymers or copolymers, salts thereof or esters thereof, polyacrylic acid/alkyl acrylate copolymers, crosslinked or non-crosslinked polyoxyethylenated AMPS/alkyl methacrylate copolymers, anionic, cationic, amphoteric or nonionic chitin or chitosan polymers, cellulose polymers other than alkylcellulose, chosen from hydroxyethylcellulose, hydroxypropylcellulose, hydroxymethylcellulose, ethylhydroxyethylcellulose, carboxymethylcellulose, and also quaternized cellulose derivatives, vinyl polymers and anionic, cationic, nonionic or amphoteric associative polymers, modified or unmodified polymers of natural origin, alginates and carrageenans, glycoaminoglycans, deoxyribonucleic acid, mucopolysaccharides, in particular hyaluronic acid and derivatives thereof, and chondroitin sulfates, and mixtures thereof;
- a lipophilic gelling agent chosen from particulate gelling agents, organopolysiloxane elastomers, semicrystalline polymers, hydrocarbon-based block copolymers, dextrin esters and polymers bearing hydrogen bonding, and mixtures thereof;
and mixtures thereof.

14. The composition as claimed in any one of claims 5 to 13, said additional ingredient being at least one compound chosen from cosmetic active agents; vitamins; UV-screening agents; dyestuffs, preferably chosen from pigments and nacres; fragrances; and mixtures thereof.

15. The composition as claimed in any one of claims 5 to 14, **characterized in that** said composition is in the form of a composition for the care and/or make-up of the skin and/or nails, of the body or face, in particular of the face, and is preferably a foundation, a face powder or an eyeshadow.

16. The composition as claimed in any one of claims 5 to 15, **characterized in that** said composition is intended for the care and/or make-up of the lips and is preferably a stick, a varnish or a gloss.

17. A process for making-up and/or caring for the skin and/or nails, comprising at least one step of applying to said skin and/or said nails a composition comprising, in a physiologically acceptable medium, at least one aqueous or aqueous-alcoholic gel of synthetic phyllosilicate of formula Mg₃Si₄O₁₀(OH)₂, having an X-ray diffraction line greater than 9.4 Å and less than or equal to 9.8 Å, said composition comprising an aqueous phase formed at least in part by said aqueous or aqueous-alcoholic gel of synthetic phyllosilicate.

18. The process as claimed in the preceding claim, in which said composition is as defined in claims 5 to 16.

19. The process as claimed in claim 17 or 18, **characterized in that** said process is directed toward affording a matt and/or homogeneous make-up result for the skin complexion after application.

20. The process as claimed in claim 17 or 18, **characterized in that** said process is directed toward facilitating the spreading of the composition on the skin and toward leading to a homogeneous deposit of active agents on the skin after application, chosen especially from cosmetic active agents; vitamins; UV-screening agents; dyestuffs, preferably chosen from pigments and nacres; fragrances; and mixtures thereof.
